# EUROPEAN PATENT SPECIFICATION

(11) **EP 2 744 913 B1**
(45) Date of publication and mention of the grant of the patent: **18.11.2015**
(21) Application number: 12753796.7
(22) Date of filing: 09.08.2012
(51) Int. Cl.: C12Q 1/68

(54) **PROGNOSTIC METHODOLOGY**
PROGNOSEMETHODOLOGIE
MÉTHODOLOGIE PRONOSTIQUE

(30) Priority: 15.08.2011 GB 201113968
(43) Date of publication of application: 25.06.2014
(73) Proprietor: University College Cardiff Consultants Limited, South Glamorgan CF24 0DE (GB)
(72) Inventor: BAIRD, Duncan, Penarth Vale of Glamorgan CF64 5DQ (GB); PEPPER, Chris, Penarth Vale of Glamorgan CF64 5RF (GB); FEGAN, Christopher, Penarth Vale of Glamorgan CF64 5BT (GB)
(74) Representative: Fyles, Julie Marie
(86) International application number: PCT/GB2012/051936
(87) International publication number: WO 2013/024264

(56) References cited:
- LIN TT; LETSOLO BT; JONES RE ET AL.: "Telomere dysfunction and fusion during the progression of chronic lymphocytic leukaemia: evidence for a telomere crisis", BLOOD, vol. 116, 2010, pages 1899-907, XP002687450, cited in the application

## Description

The invention relates to a novel prognostic method for determining at least one, or a combination, of the following: time to first treatment, response to treatment or overall survival for a patient presenting with a disease including or characterised by telomere shortening, comprising an assessment of the longest mean telomere length at which telomere end-end fusion events can be detected and then a determination of the mean telomere length in the fusogenic range (i.e. the range below said mean telomere length at which telomere end-end fusion events can be detected) and the subsequent use of the mean telomere length in the fusogenic range as a prognostic indicator. The invention also relates to the use of said method in a treatment regimen.

### Background of Invention

Chronic lymphocytic leukaemia (CLL) is the most common adult leukaemia, characterised by the accumulation of immuno-incompetent, monoclonal CD5⁺ B-lymphocytes. CLL has a very heterogeneous clinical course with survival ranging from a few months to many decades. Treatment strategies vary with staging and disease progression and include chemotherapy, radiotherapy, monoclonal antibody therapy or bone marrow transplantation, with early stage patients often receiving no treatment. Early clinical intervention is required for patients with an aggressive form of the disease, whereas patients with more benign forms simply need monitoring for disease progression at which point appropriate treatment may be administered. In this latter respect, it has been shown that early stage CLL intervention does not improve survival rates. It is therefore inappropriate to expose someone presenting with a disease that is unlikely to be life-threatening for up to 30 years with highly dangerous chemotherapeutic drugs. A reliable method for distinguishing the various forms of the disease is therefore desirable. Although the Binet and Rai staging systems are reliable predictors of clinical outcome between the staging groups, they fail to identify good and poor prognostic subsets within each stage. Since most patients present with early stage disease at diagnosis, a number of laboratory tests have been developed to try and predict the clinical course of these patients, most notably, immunoglobulin variable heavy chain somatic mutation status, CD38 expression, T-cell tyrosine kinase (ZAP-70) expression and cytogenetic abnormalities. Unmutated *IGHV* genes, high CD38 expression, high ZAP-70 expression and the presence of 17p and 11 q deletions are all associated with a poor prognosis. The exploitation of this sort of laboratory data to provide a prognostic assay is described in US 2008/0026383. However, none of these individual markers can provide definitive prognostic information alone and when used in combination offer only a reasonable prognostic prediction.

Breast cancer is another very common tumour type in the western world. Breast tumours can be surgically removed but remnants of the tumour can remain resulting in the reoccurrence of the disease. Patients therefore have adjuvant treatments that have toxic side effects, and the suspicion is that many patients receive treatment that will not be beneficial to them. The usual approach is to tailor the aggressiveness of the chemotherapy to the risk of recurrence. As compared with standard chemotherapy, aggressive chemotherapy is associated with a greater benefit, but also with more acute and long-term toxic effects such as leukaemia and heart failure. As with CLL, there is thus a requirement for markers that allow prognostication following surgery for breast cancer. Gene expression arrays have been employed to identify specific gene expression signatures that are indicative of prognosis; these provide hazard ratios of up to 3.4 for overall survival in node negative breast cancer patients. Gene expression arrays are amongst the best markers of prognostication currently available for Breast cancer.

Myelodysplastic syndromes (MDS) are a heterogeneous collection of disorders of the bone marrow haematopoietic stem cells characterised by disruption to haematopoiesis ultimately leading to bone marrow failure. This condition was previously known as 'pre-leukaemia' because one third of patients progress to acute myeloid leukaemia (AML). There is therefore a clinical need to distinguish patients that progress to AML, and thus may require therapy from those that manifest a more benign form of the disease. Like CLL, MDS is characterised by large-scale unbalanced chromosomal rearrangements; these types of rearrangements are consistent with telomere dysfunction. Furthermore, there is evidence of telomere erosion in MDS and that mutation in the telomerase RNA components can confer MDS in children.

It follows from the above, that there is a range of diseases for which relatively early stage prognostication would be advantageous. Moreover, many of these diseases are characterised by genetic abnormalities and, specifically telomere shortening. These diseases include alzheimer's disease¹, brain infarction¹, heart disease¹, chronic HIV infection¹, chronic hepatitis¹, skin diseases¹, chronic inflammatory bowel disese¹ including ulcerative colitis, anaemia¹, atherosclerosis¹, Barrett's oesophagus and cancers¹ including pre-cancerous conditions. The invention therefore has application to all of these diseases.

Telomeres are nucleoprotein structures composed of repetitive DNA sequences that cap the ends of linear eukaryotic chromosomes, protecting them from deterioration or fusion with adjacent chromosomes. During replication of DNA, the ends of chromosomes cannot be processed, and as a result during cell division the chromosome ends would be lost; telomeres however prevent this by themselves being consumed during each stage of cell division, essentially 'capping' the chromosome. Telomere ends are, however, maintained in certain cell types such as germ cells, stem cells and certain white blood cells, by the reverse transcriptase telomerase that catalyses the RNA templated addition of telomere repeats. Telomere length is a key determinant of telomeric function and it has been shown that short dysfunctional telomeres can drive genomic instability and tumourigenesis in mouse models. Furthermore, deregulation of telomerase has been shown to drive oncogenesis. Additionally, the loss of telomeres in somatic cells has been linked to replicative senescence preventing genomic instability and cancer. Conversely, it has also been shown that malignant cells can bypass this senescence and become immortalised by telomere extension by aberrant activation of telomerase.

Consistent with the role of telomere biology in tumour progression, there is now a substantial body of evidence indicating that telomere length can provide prognostic information in many human malignancies including CLL²⁻⁹. However, there is a lack of resolution in the currently available technologies and this has hampered progress in translating telomeric assays into clinical practice. For example, a putative role of telomere dysfunction during the progression of breast cancer has been shown,¹⁰ and low-resolution telomere length has been shown to provide limited prognostic information^{11,12}. A key problem with these technologies is that they are based on hybridisation of DNA probes to telomere repeat units. Consequently, as telomeres get shorter there is less probe target, and thus short telomeres are not detectable^{13,14}. This is important because it is the shortest telomeres that become dysfunctional and are subject to fusion, causing genomic instability that can drive the progression of human cancers¹⁵⁻¹⁷. Q-PCR-based methods have also been described for the estimation of telomere repeat content (WO 2004068110US), these allow for high throughput analysis. However the linearity of these methods for the detection of short telomeres (< 4 kb) has not been established¹⁸, this, coupled with the reported high CV values of up to 28%, renders the Q-PCR methods inappropriate for the detection of short telomeres and using this information as a prognostic tool for clinical decision making¹⁹. Hitherto, telomere analysis using existing low-resolution techniques is not a sufficiently informative prognostic marker.

To address this problem, we have previously developed single-molecule technologies that allow us to detect the presence of critically shortened telomeres^{20,21} and to characterise telomere end-end fusions^{16,17}. Single telomere length analysis (STELA) allows complete resolution of telomere lengths at specific chromosome ends, including telomeres in the length range in which telomere end-end fusions can occur^{16,20} It therefore permits detection of short telomeres that are potentially dysfunctional and capable of fusion. In part of this study the XpYp telomere was chosen for use in STELA because in contrast to 13q, 6q, 17p and 11q, there is no evidence to implicate the loss of this telomere in the pathology of CLL in particular. Furthermore our previous data indicate that the XpYp telomere length is representative of the genome-wide telomere length^{20,22}, and that telomerase-expressing cells can homogenise telomere lengths at different chromosome ends^{15,23}. Using these tools, we have demonstrated a link between short telomeres, telomere end-end fusion events and genomic instability in diseases such as, but not limited to, CLL breast cancer and MDS.

In our investigations, we have used telomere length and fusion analysis to provide a definition of telomere dysfunction and then we have used this as a prognostic tool. Specifically, we have identified the longest mean telomere length at which telomere end-end fusion events can be detected for a selected chromosome, examples are shown in Table 1. Using this upper limit for fusion event detection we have been able to show that the mean telomere length in the fusogenic range (i.e. ≤ the upper limit) provides a biological parameter that is highly prognostic for at least one of the following: time to first treatment, response to treatment or overall survival. Furthermore, this biological parameter can also be used to provide remarkable prognostic resolution in early stage disease patients in terms of time to first treatment, response to treatment or overall survival; indeed, patients in the longer telomere subset showed an overall survival rate of 96% at 10 years. The longest mean telomere length at which telomere end-end fusion events can be detected therefore represents an indication of the mean telomere length at which telomeres become dysfunctional and capable of fusion. Knowledge of the length of an individual's telomeres and so the likelihood of end-end fusion events enables one to predict where the individual is placed with respect to disease progression and so ensures the individual receives treatment commensurate with their requirements; no less and no more. Further, the test to assess the length of an individual's telomeres can be repeated periodically to monitor disease progression.

We have been able to show that by applying a telomere length threshold value based on telomere dysfunction, we are surprisingly able to transform the prognostic power of telomere length analysis. Thus in contrast to previous reports using low-resolution telomere length analysis (i.e. those methods described above that measure telomere length at 4kb and above), our data indicate that high-resolution telomere length analysis (i.e. using, e.g. the STELA method, or any other method which can measure the full range of telomere length from one TTAGGG repeat to over 25kb of telomere length) coupled with a definition of telomere dysfunction or a knowledge of our biological parameter, is sufficient for accurate prognostication in various diseases characterised by telomere shortening, including cancers.

### Statements of Invention

According to a first aspect of the invention there is provided a prognostic method for determining the progression of a disease or condition including or characterised by telomere shortening comprising:
i) using high-resolution telomere length analysis to determine the longest mean telomere length at which telomere end-end fusion events can be detected in samples of tissue from a number of individuals presenting with the same disease or condition, in order to identify a threshold value that represents an indication of the mean telomere length at which telomeres become dysfunctional and capable of fusion ;
ii) determining the prognostic mean telomere length of samples of tissue from a number of individuals presenting with said disease or condition, by taking those samples whose mean telomere length is less than said threshold value and averaging the mean telomere length of those samples;
iii) determining the mean test telomere length of a sample taken from a patient suspected of having or presenting with said disease or condition and, where said mean test telomere length is less than said prognostic mean telomere length it indicates that time to first treatment is poor and/or response to treatment is poor and/or overall survival is poor; or
iv) determining the mean test telomere length of a sample taken from a patient suspected of having or presenting with said disease or condition and, where said mean test telomere length is greater than said prognostic mean telomere length it indicates that time to first treatment is good and/or response to treatment is good and/or overall survival is good.

The invention therefore involves the identification of a specific methodology that permits critical telomeric parameters to be defined for a particular disease or, typically, malignancy. These parameters are the upper telomeric threshold value for end-end fusion events, as in i) above, and a subsequent prognostic mean telomere length below the said threshold value or in the fusogenic range, as in ii) above. Further, the invention also involves an analysis of patient telomere distribution, as in iii) or iv) above, and by relating this to the determined threshold value and said prognostic mean, the invention predicts whether a patient will require treatment and it also predicts progression-free or overall survival of each patient at the time the method is undertaken.

In a preferred method of the invention said fusion event in part i) above is verified as being such by direct DNA sequence analysis before the data relating to same is included in the method.

Additionally or alternatively, in a further preferred method of the invention, said prognostic mean telomere length of a sample of tissue from a number of individuals presenting with said disease is determined by taking those samples that exhibit telomere fusion and averaging the mean telomere length of those samples. This preferred method therefore includes samples whose mean telomere length is less than said threshold value and also samples whose mean telomere length is greater than said threshold value but, regardless of this fact, only samples exhibiting fusion are used to generate an average telomere length. As those skilled in the art will appreciate, the fact that the method can be worked using this additional or alternative set of samples indicates that any telomere length below said threshold value is prognostic; the mean thereof particularly so.

In a further preferred method of the invention said disease or condition including or characterised by telomere shortening comprises a disease where telomeres are shortened, as herein described, particularly where telomerase has reduced activity (statistically significant at the P<0.05 level) having regard to the average activity in immortalsied cell lines, and most preferably comprises one or more of the following diseases: ageing, alzheimer's disease; brain infarction; heart disease; chronic HIV infection; chronic hepatitis; skin diseases; chronic inflammatory bowel disease; ulcerative colitis; anaemia; atherosclerosis; Barrett's oesophagus; and cancer, including pre-cancerous conditions.

Preferably said cancer is either a haematological malignancy or a solid tumour.

Yet more preferably said cancer is CLL, MDS or breast cancer.

Yet more preferably, said telomere length at which telomere end-end fusion events can be detected is, ideally but not necessarily, determined for a selected single chromosome. Examples of chromosomes on which this analysis has been undertaken are shown in Table 1 along with the value of the upper limit for end-end fusion detection for each chromosome. Using five examples we have shown that the upper limit for detecting end-end fusion events in different chromosomes is very similar i.e. between 3.81 and 5.01 kb. The mean is 4.52kb with a standard deviation of only 0.46kb. Similarly, we have also shown that the mean telomere length in the fusogenic range for these five chromosomes is also very similar i.e. between 2.26 and 3.01 kb. The mean is 2.69kb with a standard deviation of only 0.30kb.

In an alternative preferred method of the invention, said telomere length at which telomere end-end fusion events can be detected is determined for a number of different chromosomes. Indeed, any chromosome could be used that can be subjected to high-resolution telomere length analysis. In this instance, the average upper limit for detecting end-end fusion events in the different chromosomes is used in part i) above; and the average mean telomere length in the fusogenic range for these different chromosomes in part ii) above is also used.

In a preferred method of the invention, in the case where said disease is CLL, time to first treatment is poor means an individual has a median time to treatment of less than 2 years (i.e. 1.84 years) with a hazard ratio of 23.2 indicating that they are 23.2 times more likely to require treatment in unit time than an individual with telomere length above the threshold value. Response to treatment is poor means a median time from first treatment to death of less than 5 years (i.e. 4.1 years) with a hazard ratio of 6.4 and overall survival is poor means a median survival time from diagnosis of less than 8 years (i.e. 7.49 years) with a hazard ratio of 71.3.

In a preferred method of the invention, in the case where said disease is CLL, time to first treatment is good means an individual will not need treatment and can be monitored conventionally; and response to treatment is good means that the mean time to treatment will not be reached within 10 years; and overall survival is good means that the median survival is greater than 10 years with 96% of the cohort surviving to this censor point and can be monitored conventionally.

In a preferred method of the invention, in the case where said disease is MDS, overall survival is poor means a median survival time from diagnosis of less than 1.5 years (i.e. 1.15 years) with a hazard ratio of 9.5.

In a preferred method of the invention, in the case where said disease is MDS, overall survival is good means that the median survival is 4.9 years and can be monitored conventionally.

In a preferred method of the invention, in the case where said disease is breast cancer, overall survival is poor means a median survival time of less than 1 year (i.e. 0.95 years) with a hazard ratio of 87080.

In a preferred method of the invention, in the case where said disease is breast cancer, overall survival is good means that the median survival is greater than 6 years and can be monitored conventionally.

According to a second aspect of the invention there is provided a prognostic method for determining the progression of a disease or condition including or characterized by telomere shortening comprising:
i) using high-resolution telomere length analysis to determine the prognostic mean telomere length of samples of tissue from a number of individuals presenting with said disease or condition, whose mean telomere length, the value of which has been determined according to the invention, is less than a 4.52 kb telomere length threshold value, at which telomere end-end fusion events can be detected in cancerous disease, by taking those samples whose mean telomere length is less than said threshold value and averaging the mean telomere length of those samples;
ii) determining the mean test telomere length of a sample taken from a patient suspected of having or presenting with said disease or condition and, where said mean test telomere length is less than said prognostic mean telomere length it indicates that the time to first treatment is poor and/or the response to treatment is poor and/or overall survival is poor; or
iii) determining the mean test telomere length of a sample taken from a patient suspected of having or presenting with said disease or condition and, where said mean test telomere length is greater than said prognostic mean telomere length it indicates that time to first treatment is good and/or response to treatment is good and/or overall survival is good.

In this second embodiment of the invention, preferably, said prognostic mean telomere length is determined using a 4.06kb threshold value (i.e. 4.52 - 0.46kb) or a 4.98kb threshold value (i.e. 4.52 + 0.46kb) at which telomere end-end fusion events can be detected.

In a preferred embodiment of the second aspect of the invention said disease is cancer and, typically, said cancer is CLL, breast cancer or MDS and, ideally, said prognostic mean telomere length value of 2.26kb is used for CLL and breast cancer and said prognostic mean telomere length value of 2.5kb is used for MDS.

Yet more preferably, in this second aspect of the invention said telomere length at which telomere end-end fusion events can be detected is determined for a number of chromosomes. Ideally, the chromosomes are XpYp, 17p, 2p, 16p and 18q, although any other combination of chromosomes may be used and their average upper threshold value at which telomere end-end fusion events can be detected is used in the above method.

According to a third aspect of the invention there is provided a prognostic method for determining the progression of a disease or condition including or characterized by telomere shortening comprising:
1. determining the mean test telomere length of a sample taken from a patient suspected of having or presenting with said disease or condition and, where said mean test telomere length, the value of which has been determined according to the invention is less than a prognostic mean telomere length of 2.69kb it indicates that the time to first treatment is poor and/or the response to treatment is poor and/or overall survival is poor; or
2. determining the mean test telomere length of a sample taken from a patient suspected of having or presenting with said disease or condition and, where said mean test telomere length determined according to the invention is greater than a prognostic mean telomere length of 2.69kb it indicates that the time to first treatment is good and/or the response to treatment is good and/or overall survival is good.

In this third embodiment of the invention, preferably, said prognostic mean telomere length is either 2.39kb (i.e. 2.69 - 0.3kb) or 2.99kb (i.e. 2.69 + 0.3kb).

In a preferred embodiment of the third aspect of the invention said disease is a haematological cancer, and typically said cancer is CLL or MDS and, more ideally still, said prognostic mean telomere length is 2.26kb for the former and 2.5kb for the latter.

In a preferred embodiment of the third aspect of the invention said disease is breast cancer and, more ideally still, said prognostic mean telomere length is 2.26kb.

Yet more preferably, in this third aspect of the invention said prognostic mean telomere length is determined for a number of chromosomes. Ideally, the chromosomes are XpYp, 17p, 2p, 16p and 18q, although any other combination of chromosomes may be used and their average prognostic mean telomere length is used in the above method.

According to a further aspect of the invention there is provided one or more, including combinations thereof, of the primers described herein.

According to a yet further aspect of the invention there is provided a treatment regimen including or comprising said afore prognostic method according to any aspect or embodiment of the invention.

In the claims which follow and in the preceding description of the invention, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

No admission is made that any reference cited in this specification constitutes prior art. Further, no admission is made that any of the prior art constitutes part of the common general knowledge in the art.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

The invention will now be described by way of example only with reference to the following tables and figures:
Table-1 shows the longest mean telomere length at which telomere end-end fusion events can be detected for a range of chromosomes, including the mean thereof and the prognostic mean telomere length for each one of said chromosomes, including the mean thereof.
Table-2 shows a comparison of prognostic factors in univariate analysis, in terms of time to first treatment and overall survival.
Table-3 shows the clinical characteristics of the 184 CLL patient cohort.
Table-4 shows the analysis of concordant datasets combining telomere length analysis with known prognostic markers.
Figure-1 defines the telomeric parameters for prognosis in CLL. [A] An example of STELA at the XpYp telomere in 12 CLL patients in which fusion was, or was not detected. Mean and standard deviation are displayed below and the means highlighted in red on the gel image. [B] Examples of fusion analysis in 4 CLL patients. [C] Examples of the DNA sequence of the fusion events highlighted in panel B. Arrows indicate the fusion junction, together with the participating telomere and the deletion from the start of the respective telomeres. Homology between the participating telomeres is underlined. [D] Mean XpYp telomere length data plotted as a function of Binet staging. Black markers indicate those that were not tested for fusion, blue those that were negative and red those that were positive for fusion events. Panel E shows telomere length data from the whole cohort, together with those that were positive for fusion events. The longest mean XpYp telomere (3.81 kb) in which fusion was detected is indicated with a dashed line and mean XpYp telomere length of the samples in which fusion was detected was 2.26kb.
Figure-2. Mean telomere length is prognostic in CLL. Panels A and B show Kaplan Meier curves from the entire cohort for time to first treatment upper graph and overall survival lower graph. P values, Hazard Ratio (HR) are indicated on the plots together with numbers in each arm.
Figure-3 shows that telomere length, as defined by fusion, is highly prognostic in CLL. [A-B, E] Kaplan Meier curves from the entire cohort, for time to first treatment and overall survival. P-values and Hazard Ratio (HR) are indicated on the plots, together with numbers in each arm. [C-D] Kaplan Meier curves for the Binet stage A only cohort. [F-G] Recursive partitioning of the data set shows 2.26kb is the optimal telomere threshold value as a prognostic tool for defining survival in the whole data set, and in the 2 population cohorts.
Figure-4. Panel A shows mean 17p telomere length data plotted as a function of Binet staging. Black markers indicate those that were not tested for fusion, blue those that were negative and red those that were positive for fusion events. Panel B shows telomere length data from the whole cohort, together with those that were positive for fusion events. The longest mean XpYp telomere (4.81 kb) in which fusion was detected is indicated with a dashed line and denotes the upper limit of the fusogenic range for the 17p telomere. The mean telomere length of the samples in which fusions could be detected was 2.57kb. Panels C and D show Kaplan Meier curves for time to first treatment and overall survival respectively based on a cut-off of 2.5kb derived from recursive partitioning of the data. Panels E and F show Kaplan Meier curves for time to first treatment and overall survival respectively for stage A patients only based on a cut-off of 2.5kb derived from recursive partitioning of the data. Panel G shows a plot of mean telomere length of the 17p telomere versus hazard ratios for overall survival. Recursive partitioning illustrates that 2.5kb is the optimal threshold value for defining prognosis using this telomere.
Figure-5. shows that telomere length is superior to other known prognostic parameters. Kaplan Meier curves with telomere length together with cytogenetics [A-B], *IGHV* status [C-D], CD38 status [E-F] and ZAP-70 status [G-H] as a function of both time to first treatment and overall survival.
Figure-6. Shows that the telomere threshold value of 2.26kb, derived from the XpYp chromosome, is highly prognostic for CLL patient response to treatment. Kaplan Meier curves for a subset of patients with CLL that received treatment (n = 75). Survival time was calculated from time of first treatment. P-values and Hazard Ratio (HR) are indicated on the plots, together with numbers in each arm.
Figure-7. Shows that telomere length, as defined by fusion, is also prognostic in breast cancer. [A-D] Kaplan Meier curves from the entire cohort, for overall survival. P-values and Hazard Ratio (HR) are indicated on the plots, together with numbers in each arm. [E] Recursive partitioning of the data set shows that 2.26kb is the optimal telomere threshold value as a prognostic tool for defining survival in the whole data set.
Figure-8 MDS figure shows that the 2.26kb telomere threshold value offers limited prognostic power in MDS. [A-D] Kaplan Meier curves from the entire cohort, for overall survival. P-values and Hazard Ratio (HR) are indicated on the plots, together with numbers in each arm. D shows the 2.5kb telomere threshold value offers better prognostic power in MDS. [E] Recursive partitioning of the data set shows that 2.5kb is the optimal telomere threshold value as a prognostic tool for defining survival in the whole data set.

### METHODS

### CLL Patients

Peripheral blood samples from 184 CLL consenting patients, in accordance with the Declaration of Helsinki and as approved by the South East Wales local research ethics committee (LREC# 02/4806). CLL was defined by clinical criteria as well as cellular morphology, and also the co-expression of CD19 and CD5 in lymphocytes simultaneously displaying restriction of light-chain rearrangement. Comprehensive clinical information was available for all patients with a median follow-up of 5.8 years. All of the samples were collected at, or close to, the time of diagnosis from two centers, Cardiff and Birmingham, and staging was based on the Binet classification system²⁴. The clinical characteristics of the CLL patient cohort are presented in Table-2.

### Breast cancer Patients

Genomic DNA, together with clinical follow up data, from a panel of 28 invasive breast ductal carcinomas was obtained from the Wales Cancer bank, under approval from the Wales MREC.

### MDS Patients

Bone marrow samples were obtained from 63 patients diagnosed with myelodysplastic syndrome (MDS), as classified according to the French-American-British system. Of these, 40 patients were male and 23 were female, with a mean age at diagnosis of 67.5 years; the median follow-up for the cohort was 5.6 years. IPSS criteria were available for 55/63 patients with 15 high, 20 intermediate and 20 low.

### Isolation of peripheral blood mononuclear cells from CLL patients

Peripheral blood mononuclear cells (PBMCs) were isolated from EDTA venous blood of the 184 CLL patients by density centrifugation using Ficoll-Hypaque (Invitrogen). B-cells were subsequently positively isolated using CD19-labeled Dynabeads (Invitrogen)²⁵. Cells were stored at -20°C as dry pellets prior to DNA extraction.

### DNA extraction and PCR

DNA was extracted from human cells using standard proteinase K, RNase A, phenol/chloroform protocols²⁶. For telomere length analysis at the XpYp, 17p, 2p, 16p and 18q telomeres, we used a modification of the single telomere length analysis (STELA) assay as previously described^{16,20}. Briefly, genomic DNA was solubilized by dilution in 10mM Tris-HCl (pH 7.5), quantified by using Hoechst 33258 fluorometry (BioRad, Hercules, USA), and diluted to 10ng/µl in 10mM Tris-HCl (pH 7.5). DNA (10ng) was further diluted to 250 pg/µl in a volume of 40µl, containing Telorette2 linker (1µM) and Tris-HCl (1 mM; pH 7.5). Multiple PCR reactions (typically 6 reactions per sample) were carried out for each test DNA, in 10µl volumes. The reaction mixture consisted of DNA (250pg), telomere-adjacent and Teltail primers (0.5µM), Tris-HCl (75mM; pH8.8), (NH₄)₂SO₄ (25mM), 0.01% Tween-20, MgCl₂ (1.5mM), and 0.5 U of Taq (ABGene, Epsom, UK) and Pwo polymerase (Roche Molecular Biochemicals, Lewes, UK) in a 10:1 ratio. The reactions were cycled with an MJ PTC-225 thermocycler (MJ research, Watertown, USA). The DNA fragments were resolved by 0.5% TAE agarose gel electrophoresis, and detected by two separate Southern hybridizations, with random-primed α-33P labeled (Amersham Biosciences, Little Chalfont, UK) TTAGGG repeat probe and a telomere-adjacent probe, together with a probe to detect the 1 kb (Stratagene, La Jolla, USA) and 2.5 kb (BioRad) molecular weight marker. The hybridized fragments were detected by phosphorimaging with a Molecular Dynamics Storm 860 phosphorimager (Amersham Biosciences, Little Chalfont, UK). The molecular weights of the DNA fragments were calculated using the Phoretix 1D quantifier (Nonlinear Dynamics, Newcastle-upon-Tyne, UK).

Telomere fusion was detected using the previously described single molecule telomere fusion assays^{16,17}. PCR reactions containing 100ng of DNA were performed, each containing the XpYpM, 17p6 and 21 q1 PCR primers. Fusion molecules were detected, and the frequencies quantified by Southern blotting and hybridization with the XpYp telomere-adjacent probes as described previously¹⁵. In order to determine the chromosomes participating in the fusion events for subsequent sequence characterization, further hybridisations were undertaken with the 17p and 21 q telomere adjacent probes; the 21 q probe yields additional nonspecific products and thus was not used for quantification. Any fusion products were then re-amplified for direct sequence analysis using nested PCR primers (XpYpO, 17p7 and 21qseq1).

The oligonucleotides utilised were: XpYpM (5'-ACCAGGTTTTCCAGTGTGTT-3'), 17p6 (5'-GGCTGAACTATAGCCTCTGC-3'), 21 q1 (5'-CTTGGTGTCGAGAGAGGTAG-3') for fusion PCR; XpYpO (5'-CCTGTAACGCTGTTAGGTAC-3'), 17p7 (5'-CCTGGCATGGTATTGACATG-3'), 21qseq1 (5'-TGGTCTTATACACTGTGTTC -3') for re-amplification of fusion products; 21qseq1 (5'-TGGTCTTATACACTGTGTTC -3'), 21qseq1rev (5'-AGCTAGCTATCTACTCTAACAGAGC-3'), XpYpO (5'-CCTGTAACGCTGTTAGGTAC-3'), XpYpB2 (5'-TCTGAAAGTGGACC(A/T)ATCAG-3'), 17p7 (5'-CCTGGCATGGTATTGACATG-3'), 17pseq3 (5'-AGAATCCTGTCCTCAACAAGT-3') to generate hybridisation probes for fusion analysis.

Primers that can be used for STELA analysis (the ones that are typically used **emboldened):**
**XpYpE2 TTGTCTCAGGGTCCTAGTG**
**XpYp-427A/415T GGTTATCAACCAGGTGCTCT**
**XpYp-427G/415C GGTTATCGACCAGGTGCTCC**
XpYpins TGTGTCTGGAATTGGTGGGTT
XpYpdel CCTAGTGTGTCTGGAATTGGTTC
XpYpM ACCAGGTTTTCCAGTGTGTT
XpYpC CAGGGACCGGGACAAATAGAC
XpYpO CCTGTAACGCTGTTAGGTAC
**17pseq1rev GAATCCACGGATTGCTTTGTGTAC**
17p6 GGCTGAACTATAGCCTCTGC
17p7 CCTGGCATGGTATTGACATG
**16prev1 GTGAATAATCAAGGTCAGAGCA**
**18qrev4 CCTGTGGGTCTAAAACCAGAAGG**
**2p2 GAGCTGCGTTTTGCTGAGCAC**
**11q13B CAGACCTTGGAGGCACGGCCTTCG**
**12q-197A GGGAGATCCACACCGTAGCA**
**12q-550C ACAGCCTTTTGGGGTACCGC**

### Statistical methods

Statistical analysis was carried out using Prism 3.0 (Graphpad) and SAS version 9.1.3 software (SAS Institute).

The relationship between telomere length, known prognostic factors, time to first treatment (TTFT) and overall survival (OS) were explored through Wilcoxon rank sum tests for the categorical variables Binet stage, CD38, ZAP-70, *IGHV* gene mutation status, β2-microglobulin and FISH cytogenetics. Unstratified univariate comparisons of survival between the prognostic subsets were conducted with the log-rank test, with survival data displayed using Kaplan-Meier curves. Multivariate analysis, which adjusted for other prognostic features, was performed using forward selection to define significant co-variables with Cox regression. A P-value < 0.05 was considered significant.

### RESULTS

### Telomere length and fusion analysis

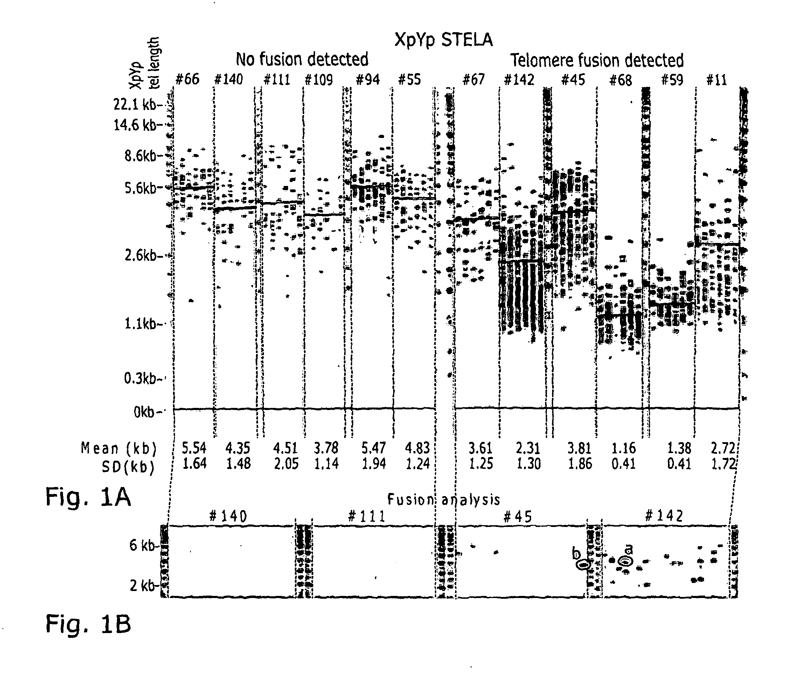

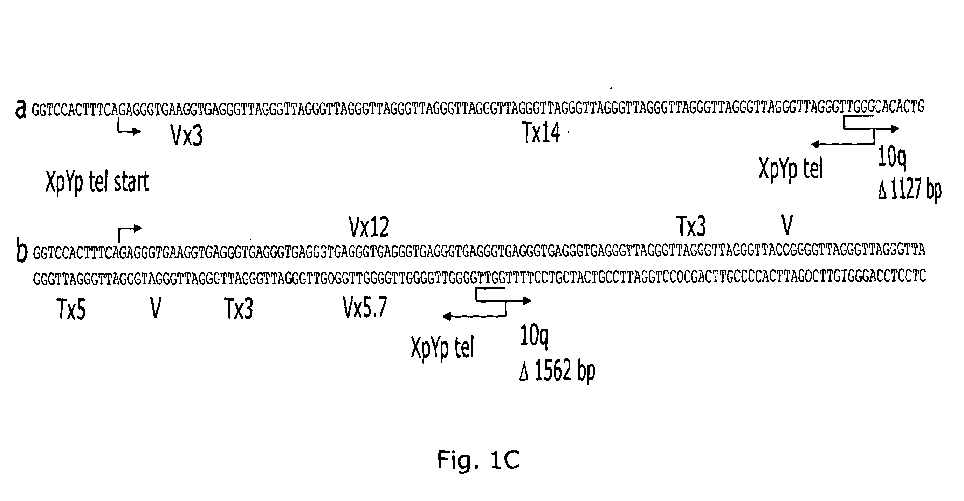

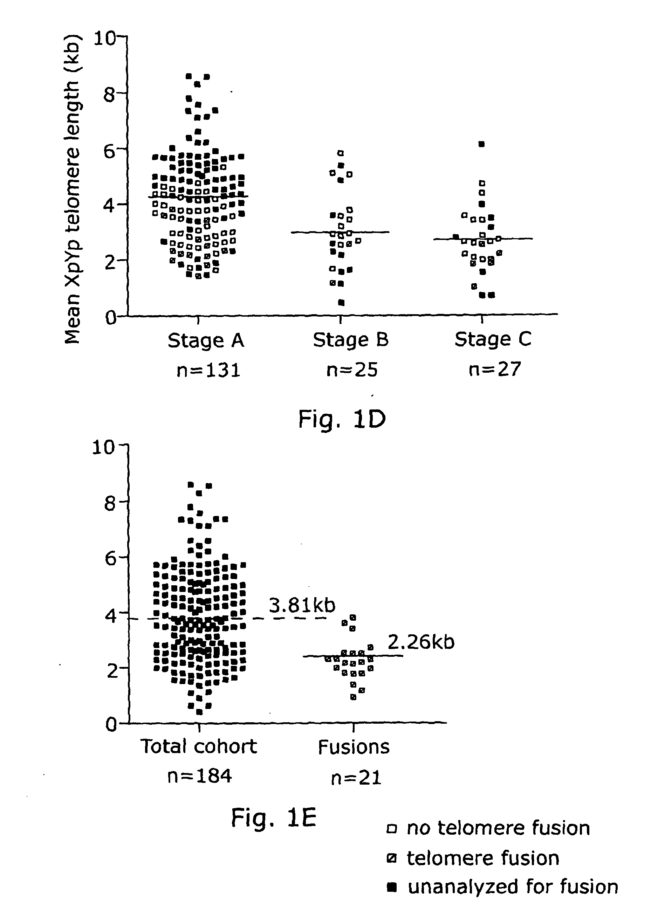

We analyzed the telomere length distribution in 184 CLL patients using single telomere length analysis (STELA) at the XpYp telomere (Figure 1A). Given that we have previously shown that telomere end-end fusion events can be detected in CLL patients with short telomeres¹⁵, we systematically looked for telomere fusions in the CLL samples with the shortest mean telomere lengths (n = 88). We only considered a fusion event to be *bone fide* when it could be fully characterized by direct DNA sequence analysis (Figure 1 B, 1 C). Telomere fusions were detectable in samples derived from all Binet stages, suggesting that they are not merely a characteristic of advanced disease (Figure 1 D; fusions marked in red). However, fusions were only detected in samples with a mean telomere length of ≤3.81kb. We therefore used this telomere length as a threshold value to define the upper limit of the 'fusogenic' range for our cohort using this chromosome. Figure 1 E shows that 98/184 (53.3%) of the CLL samples had a mean XpYp telomere length equal or less than 3.81 kb with a mean fusogenic telomere length of 2.26kb. We therefore used 2.26kb as a way of defining two subsets of CLL patient samples in our cohort and determined the prognostic value of this mean telomere length threshold value in our cohort. A total of 33/184 (17.9%) of the samples had a mean telomere length ≤2.26kb.

### Telomere dysfunction is highly prognostic in CLL

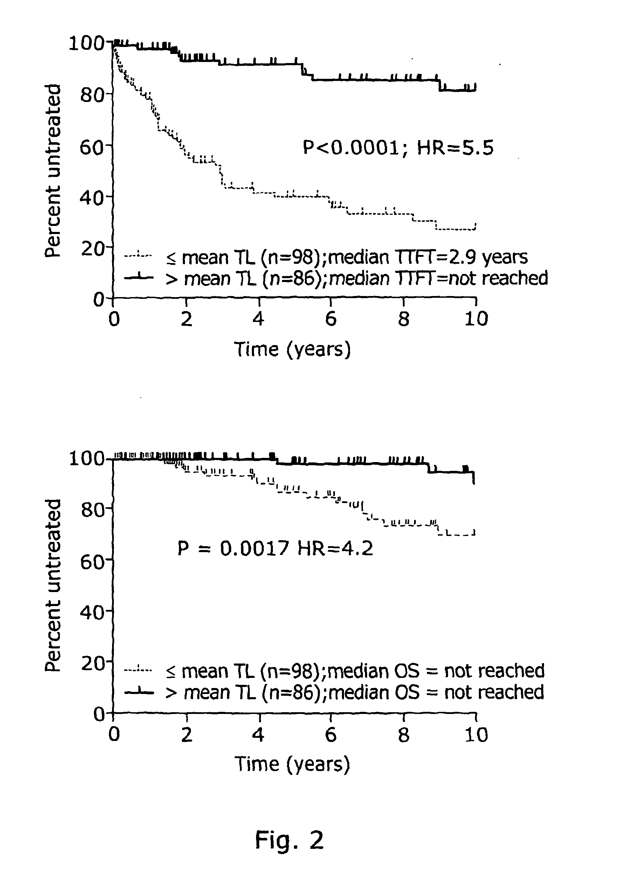

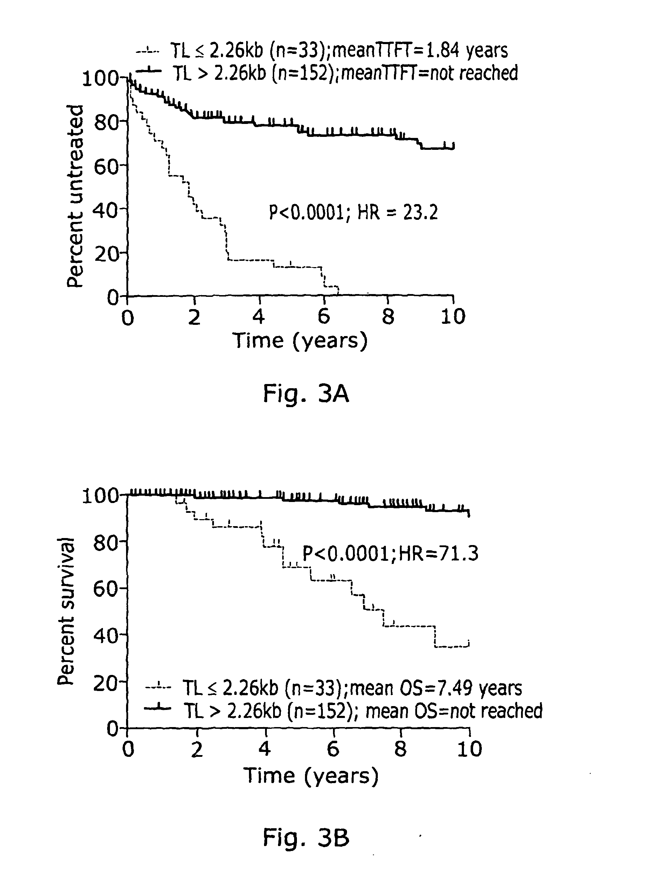

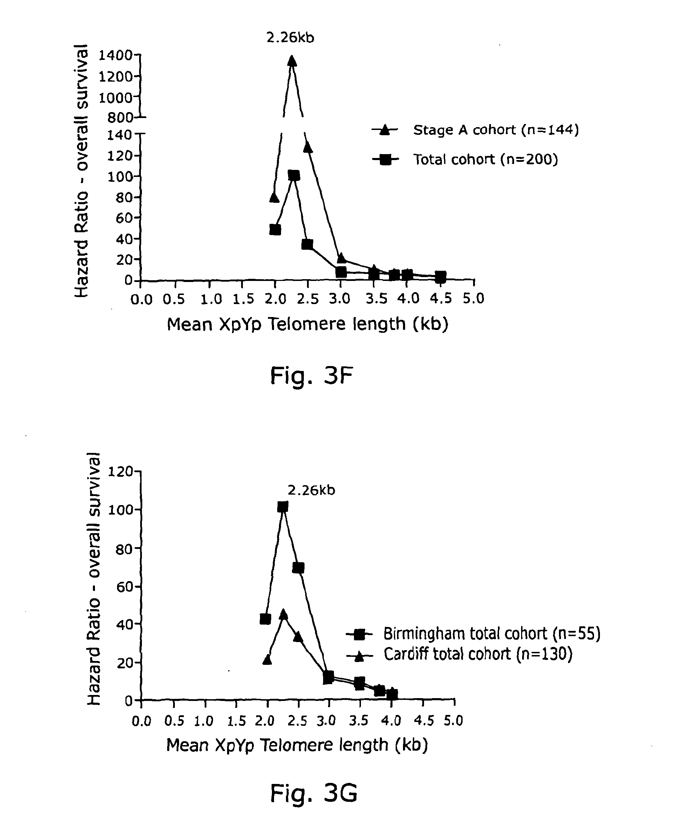

In keeping with previous studies, mean telomere length was prognostic in our cohort of patients for TTFT (P<0.001; HR=5.5) and OS (p=0.0017; HR4.2) (Figure 2). However, categorization of the samples based on telomere dysfunction (≤2.26kb telomere length of the XpYp telomere) revealed remarkably enhanced prognostic discrimination. Figure 3A and 3B show that a mean telomere length ≤2.26kb) was highly prognostic for TTFT and OS. The median TTFT was 1.8 years (P<0.0001; HR = 23.2) and the median OS was 7.5 years (P<0.0001; HR = 71.3). In contrast, the median TTFT and OS were not reached in the longer telomere subset. Particularly striking was the impact of telomere length on OS in our cohort; the Kaplan Meier curve for patients with >2.26kb telomere length showed almost no erosion over the 10 year follow-up period; 98% survival at 5 years and 96% survival at 10 years. Whereas, only 36% of the short telomere group was alive at the 10-year censor point indicating that patients with ≤2.26kb were more than 70 times more likely to die in unit time. These data are summarised in Table-2.

### Stage A patients with short telomeres have more aggressive disease

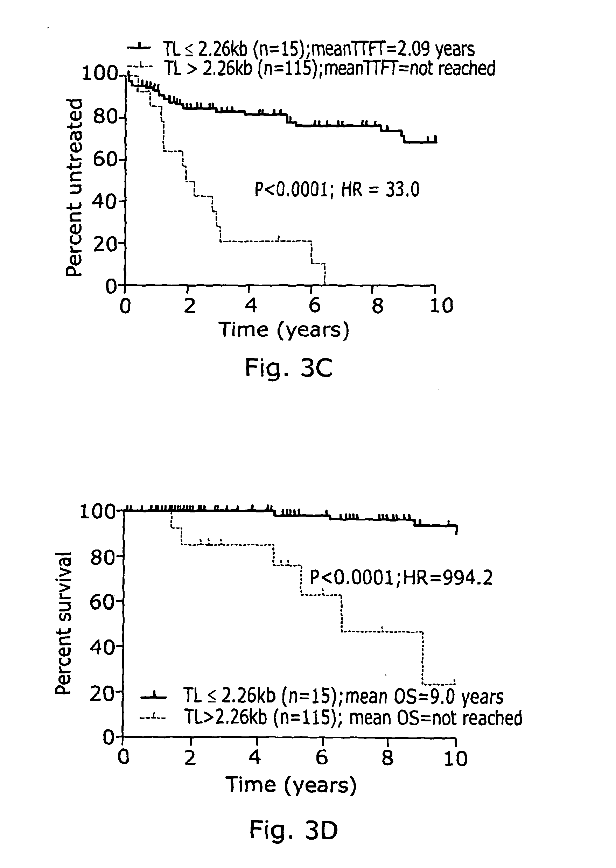

Given that the majority of CLL patients present with early stage disease and this group represent the greatest challenge in terms of prognostication, we performed a subset analysis of only the Binet stage A patients. 130/184 (70.6%) of our cohort was Binet stage A at diagnosis of which 15 (11.5%) had ≤2.26kb) telomere length for the XpYp telomere. Figures 3C and 3D show the prognostic impact of short telomeres in early stage disease. The median TTFT was 2.1 years (P<0.0001; HR = 33.0) and the median OS was 9.0 years (P<0.0001; HR = 994.2). Once again, the median TTFT and OS were not reached in the longer telomere group. The remarkable hazard ratio for OS suggests that these patients are almost 1000 times more likely to succumb to their disease in unit time than patients with longer telomeres. Once again, the superior Kaplan Meier curve revealed that patients with longer telomeres had a survival rate of 96% at 10 years.

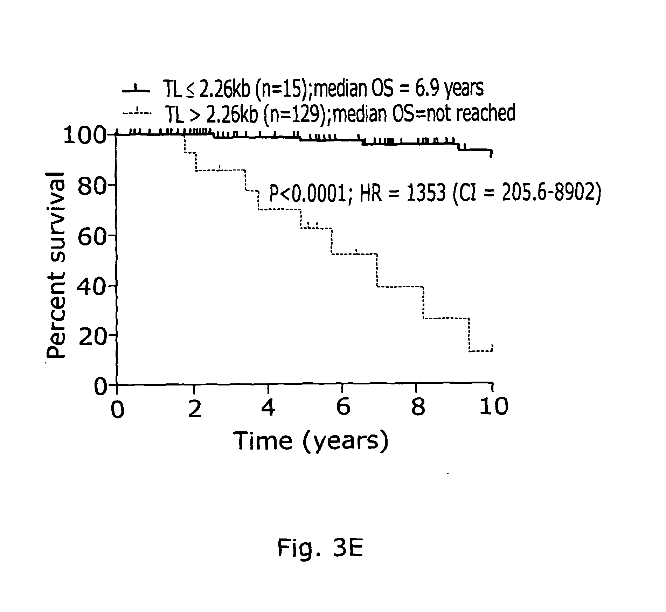

Expansion of the dataset to 144 Stage A patients, provided further verification that the specific telomere length of 2.26 kb provided the maximal prognostic power for this assay in CLL and the HR for overall survival increased to 1353 (Figure 3E).

### Recursive partitioning identifies the 2.26kb threshold value as most prognostic for survival

Although we had experimentally determined the telomere length for telomere dysfunction in CLL and shown that this was highly prognostic, we wanted to establish if this represented the optimal telomere length cutoff for predicting survival in our cohort. By performing recursive partitioning on our data set, we found 2.26kb represented the optimum telomere length, and was the most prognostic threshold value for the total cohort and the Stage A cohort (Figure 3E). Given that our cohort was made of samples derived from two different centers (Cardiff and Birmingham), we repeated the analysis in these two separate populations and derived essentially the same result (Figure 3F). This approach provides further circumstantial evidence that 2.26kb represents the biological limit of telomere stability and confirms the clinical importance of this mean fusogenic telomere length in CLL.

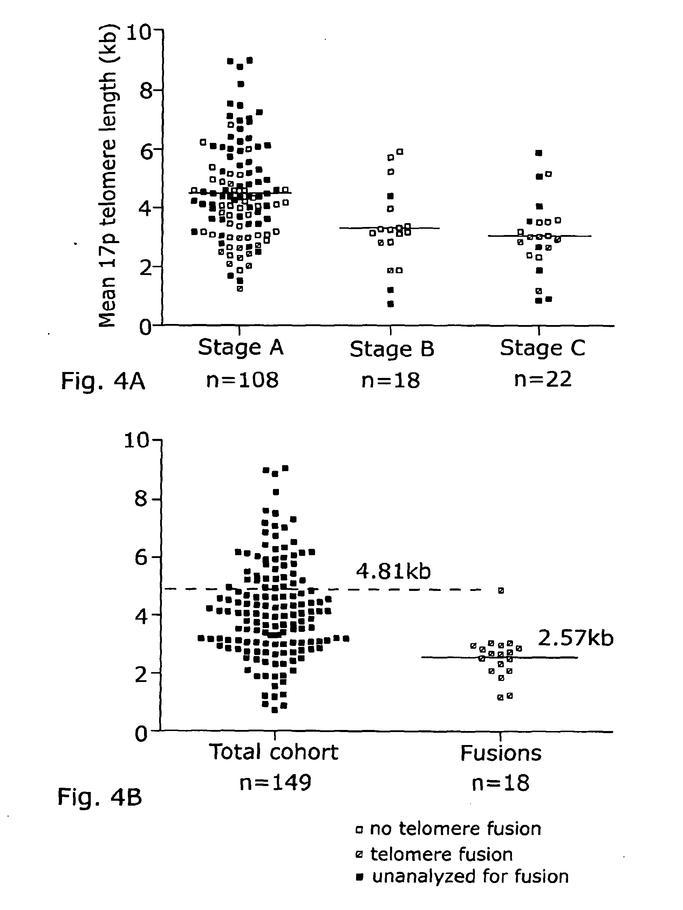

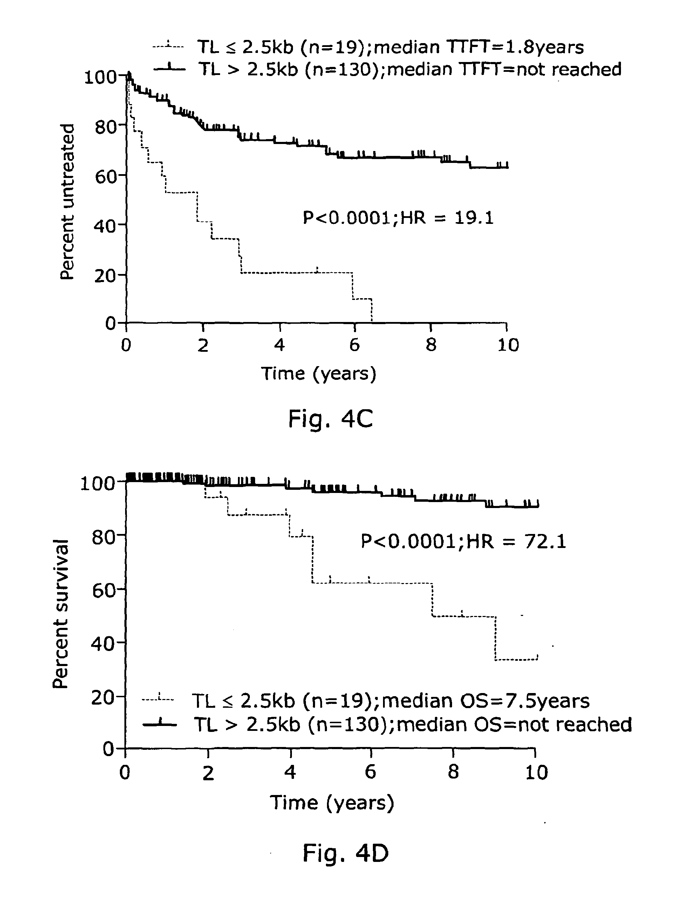

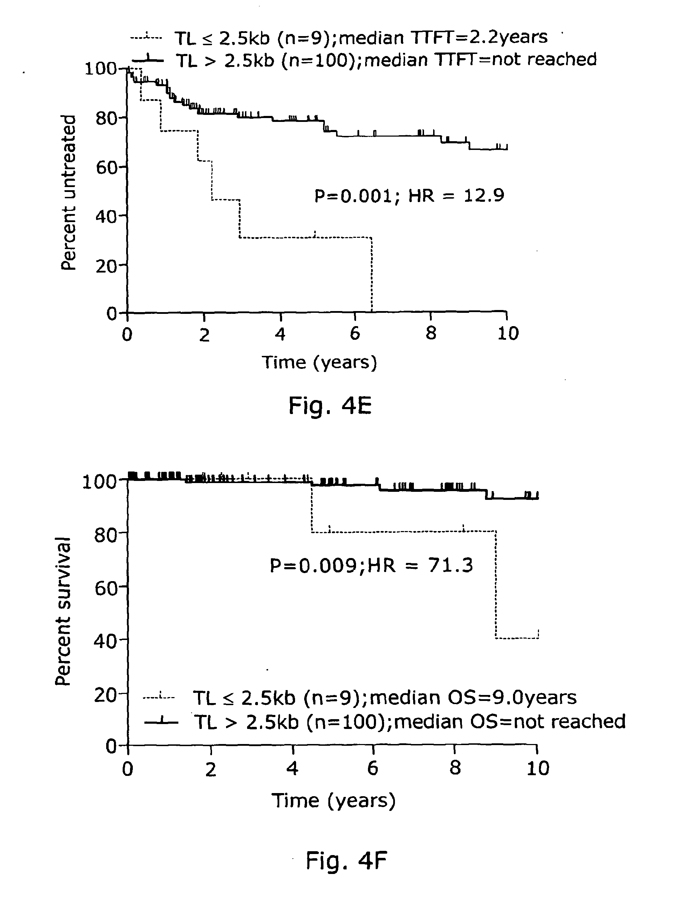

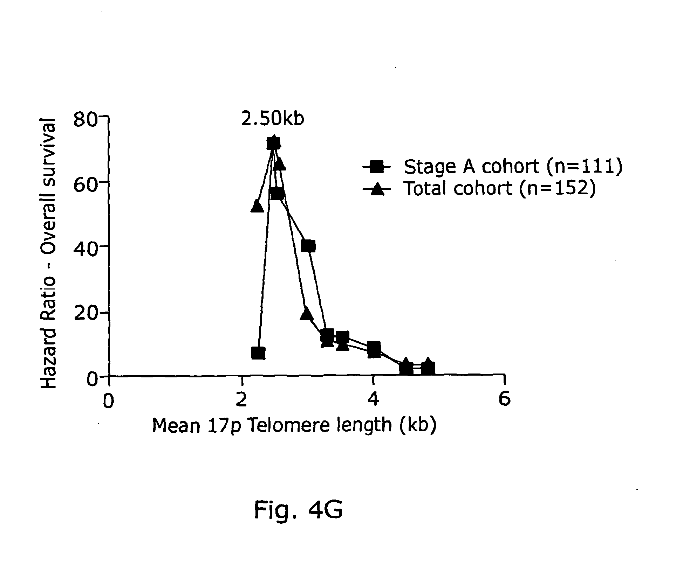

We considered that this mean fusogenic telomere length may be conserved at other chromosome ends and thus we analyzed telomere length at 17p (Figure 4A) in 149/184 (81%) of the patient cohort. The mean 17p telomere length of the samples in which we could detect fusions was similar to that observed at XpYp (2.57kb, ±0.79, P = 0.21; Figure 4B). Recursive partitioning revealed the optimal telomere length for determining prognosis was 2.5kb; this was highly prognostic in the whole cohort (OS P<0.0001, HR = 72) and stage A patients (OS P = 0.009, HR = 71, Figure 4C-G).

### Telomere length is superior to other prognostic parameters

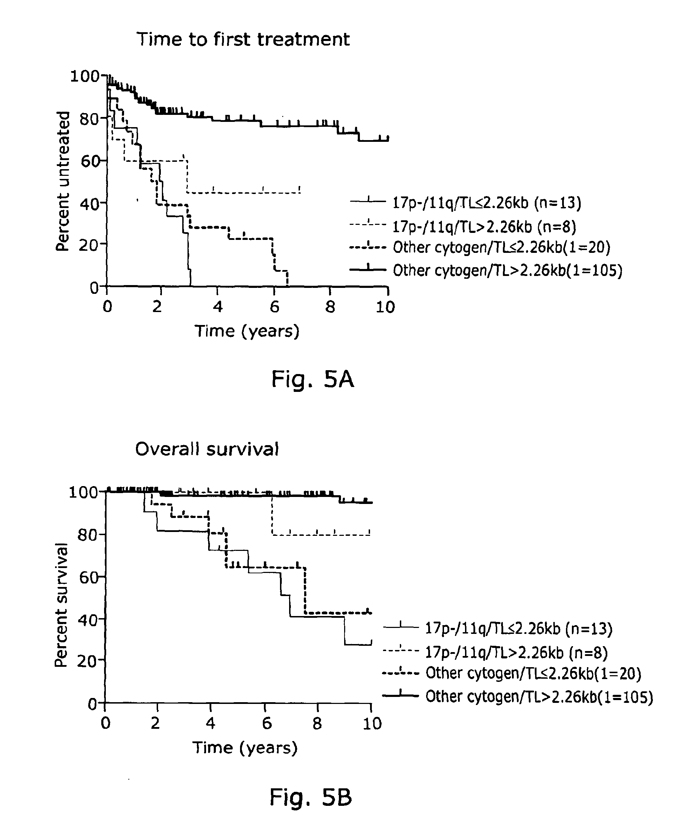

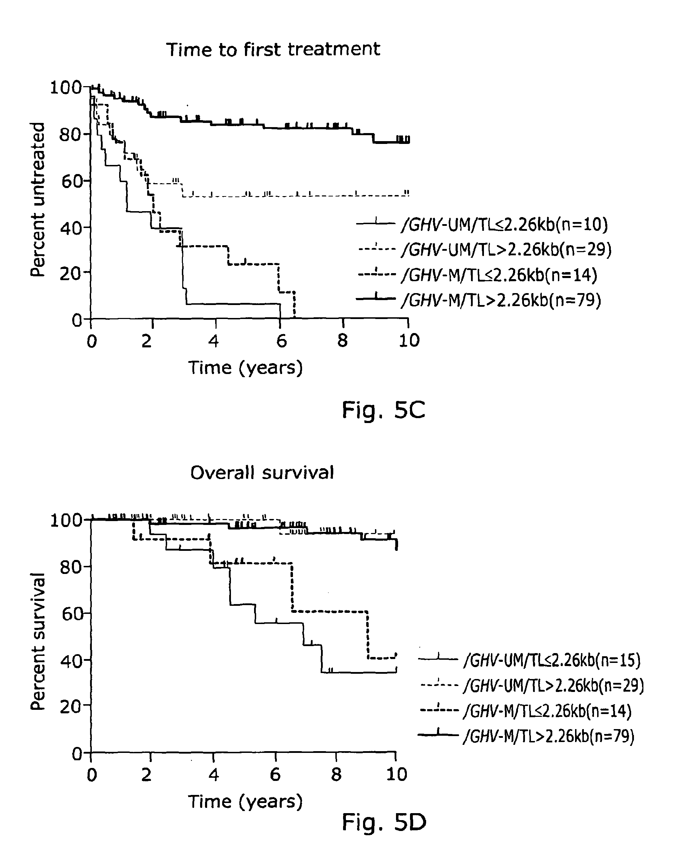

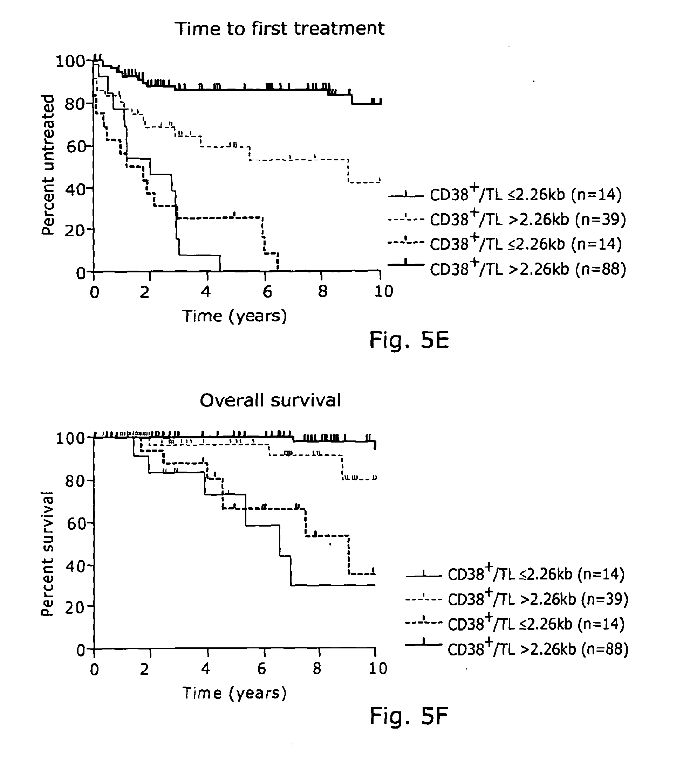

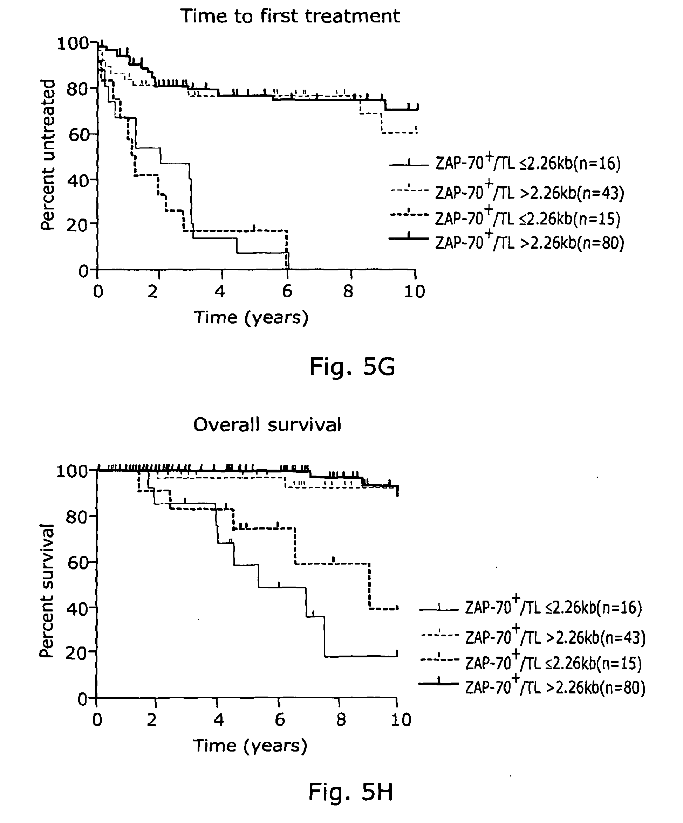

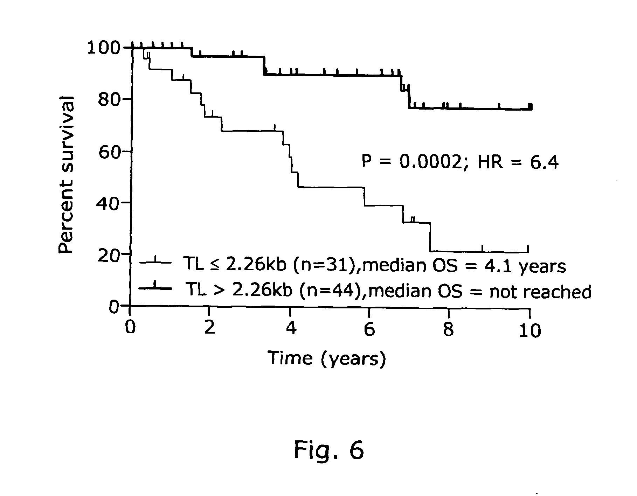

We next investigated the impact of dysfunctional telomeres on other known prognostic markers in CLL, including cytogenetics, *IGHV* mutation status, CD38 expression, ZAP-70 expression and Beta-2 microglobulin (β2M). The combined analysis of telomere length with FISH cytogenetics, *IGHV* mutation status, CD38 expression, ZAP-70 expression are shown in Figure 5. As shown, short telomere length defines poor prognostic subsets of patients within cytogenetic risk groups, *IGHV* unmutated and mutated groups, CD38⁺ and CD38- groups, ZAP-70⁺ and ZAP-70- groups and β2M high and low groups, in terms of TTFT and OS. Combining these markers with telomere length enhanced the prognostic power still further; for example, the analysis of the concordant datasets revealed that high CD38 expression in conjunction with telomere length ≤2.26kb yielded a HR of 2915 (P<0.0001, Table-4).

### Telomere length is the dominant co-variable in multivariate analysis

In multivariate analysis forward selection identified telomere dysfunction (≤2.26kb) as the most significant parameter for TTFT (HR = 4.2; CI 1.9-8.8, P = 0.0002) and OS (HR = 10.9; CI 3.8-31.2, P<0.0001). Only *IGHV* mutation status and Binet stage retained independent prognostic significance as co-variables in the model for TTFT and only CD38 in terms of OS. It is of particular interest that *IGHV* mutation status and 'high-risk' cytogenetics were not independently prognostic in terms of OS. To our knowledge, this is the first time that these parameters have failed to prove significant for OS in this disease.

### Telomere length defines response to treatment in CLL

Given that we have shown that telomere length provides powerful prognostic information in CLL, we further considered that telomere length may also provide information about the ability of patients to respond to treatment. We therefore undertook a subset analysis (n=75) of our CLL patient cohort for those that had received treatment. Telomere length was highly prognostic for response to treatment with a HR of 6.4 (P = 0.0002) (Figure-6).

### Telomeric parameters defined in CLL are prognostic in other indications.

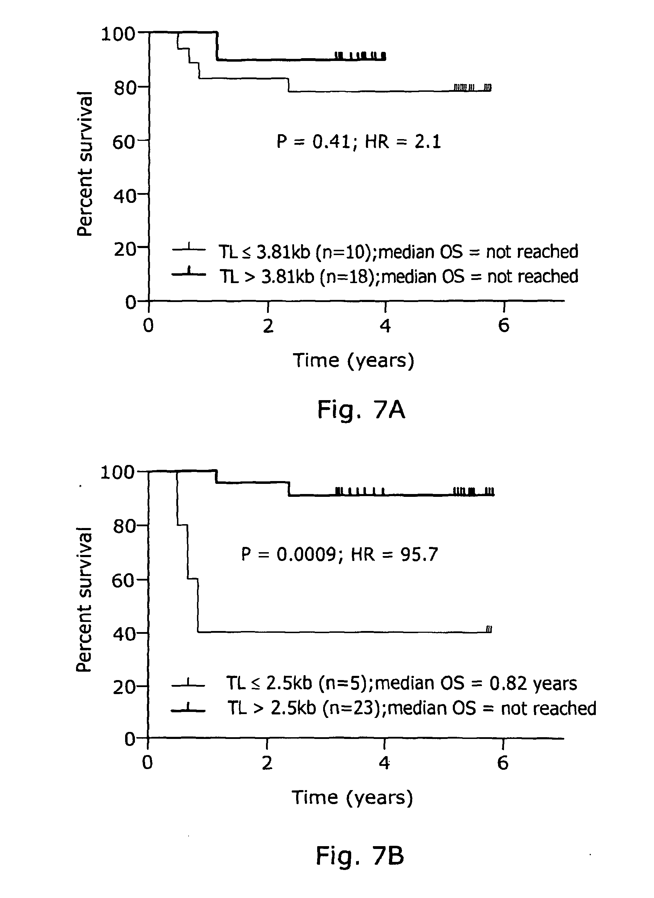

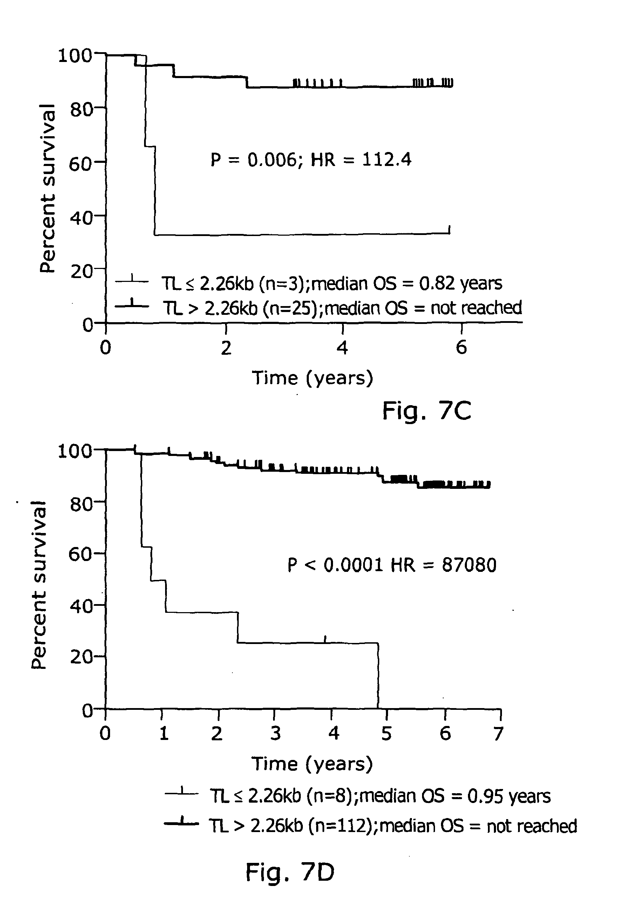

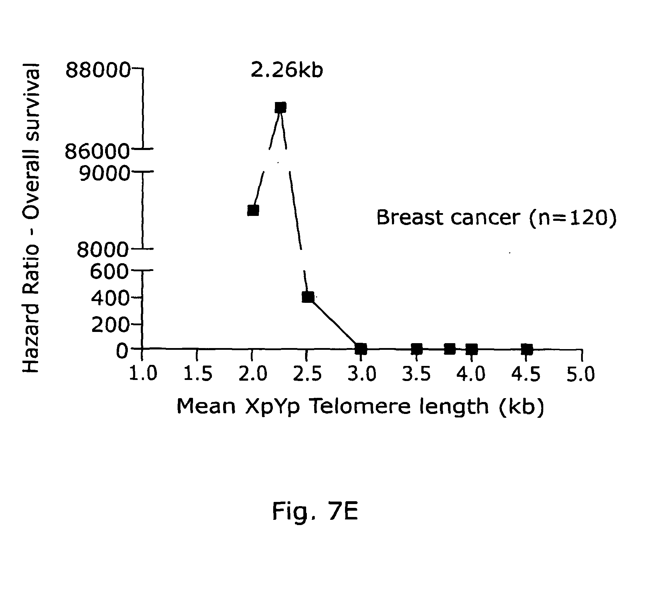

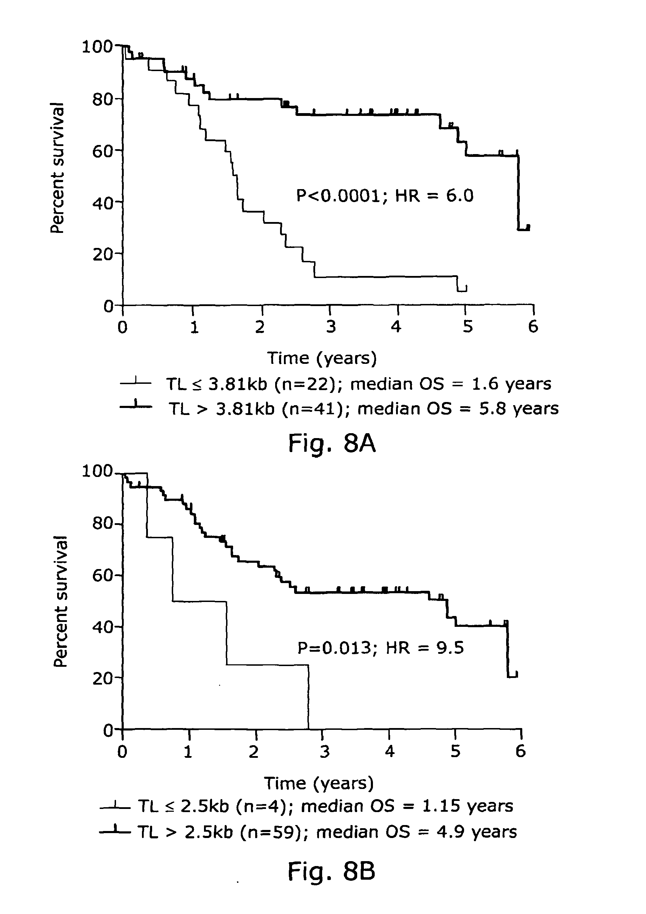

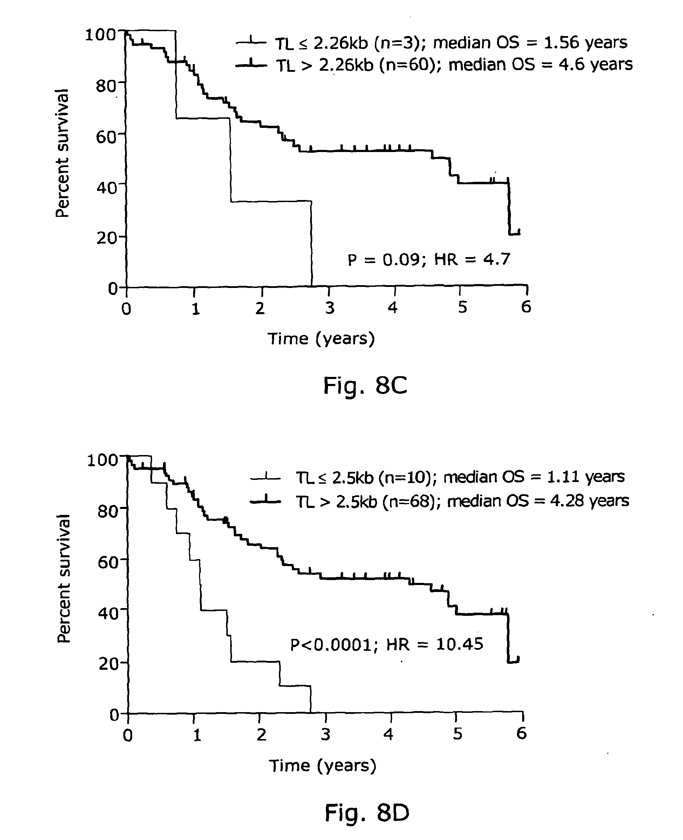

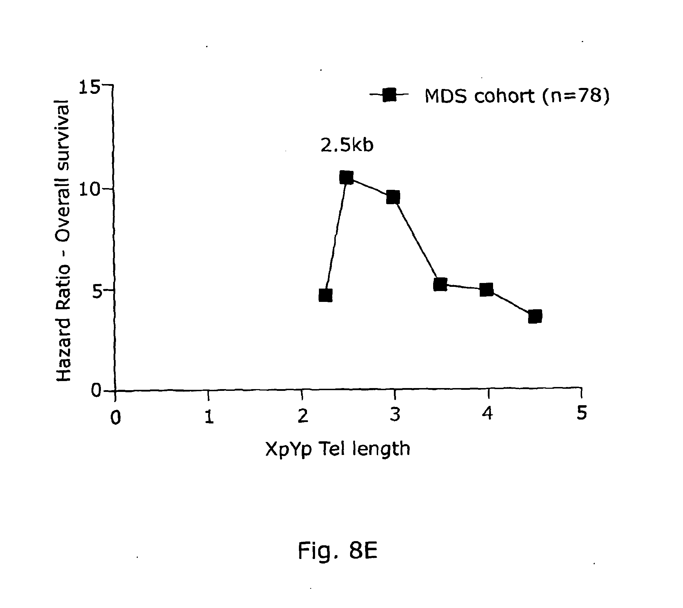

We examined a cohort of 28 patients with invasive ductal carcinoma of the breast. We analyzed XpYp telomere length using STELA and categorized the patients based on the 2.26kb telomere length cutoff defined in CLL. Despite a limited follow up period of 4.6 years, the 2.26kb mean fusogenic telomere length provided remarkable levels of prognostication for overall survival in this disease with a hazard ratio of 112 (P = 0.0056), and a median survival in the poor prognostic group of 301 days (Figure-7A-C). Expansion of the dataset to 120 breast cancer patients, provided further verification that the specific telomere length of 2.26 kb provided the maximal prognostic power for this assay in breast cancer and the HR for overall survival increased to 87080 (Figure 7D).

As with CLL, recursive partitioning of the Breast Cancer Cohort data showed that the optimum telomere length as defined by HR was 2.26kb (Figure-7E).

We also examined telomere length in MDS using STELA and used the mean fusogenic telomere length defined in CLL to provide prognostic information in MDS. We analysed a panel of 63 MDS patients for which we had survival data. The 2.26kb mean fusogenic telomere length as defined in CLL, provided some prognostic power in MDS with a HR of 4.7 (P = 0.09) for overall survival (Figure-8A-C). Unlike the CLL and breast cancer samples, the MDS samples were not purified and contained varying unidentified proportions of unaffected cells. We considered that the presence of unaffected normal cells would skew the optimal telomere length threshold value for prognostication in this cohort. This was apparent from the recursive partitioning, where the optimal telomere length was 2.5kb (HR = 9.5, P = 0.026) a difference of just 240 bp (Figure-8E). Expansion of the dataset to 78 MDS patients provided further verification that the specific telomere length of 2.5 kb provided the maximal prognostic power of this assay in MDS and the HR for overall survival increased to 10.45 (Figure 7D). Purification of MDS cells using CD34 may improve the accuracy of telomere-based prognostication in MDS.

### Summary

The main findings of this study can be summarised as follows:
Telomere length analysis, as defined by telomere dysfunction, provides a highly prognostic tool in human diseases, such as CLL and other human malignancies, permitting considerable discrimination for clinical outcome following treatment. Prognostic power should enable clinicians to confidently predict the clinical course of these heterogeneous diseases.

Moreover, telomere dysfunction provides remarkable prognostic resolution in early disease stage.

Only telomeres in the lower portion of the length distribution profile have the propensity for end-end fusion; using the XpYp chromosome a telomere length of ≤2.26kb is a mean fusogenic telomere length for telomere dysfunction in a primary human tumor, below which patients of human malignancies show poor prognostic outcome. Using a number of chromosomes a telomere length ≤2.69kb is a predictor for telomere dysfunction.

Patients with XpYp telomeres longer than 2.26kb have remarkably stable and indolent disease (98% of these patients were alive at 5 years and 96% at the 10-year censor point).

Consistent telomere analysis in MDS and breast cancer shows that high-resolution telomere length analysis is likely to be highly prognostic in other haematological malignancies but importantly also in solid tumours.

By applying a telomere length threshold value based on telomere dysfunction, this transforms the prognostic power of telomere analysis into the most prognostic parameter ever described in both univariate and multivariate analysis.

### References

1. Jiang H, Ju Z, Rudolph K L. Telomere shortening and ageing. Z. Gerontol Geriat 2007; 40:314-324.
2. Gertler R, Rosenberg R, Stricker D, et al. Telomere length and human telomerase reverse transcriptase expression as markers for progression and prognosis of colorectal carcinoma. J Clin Oncol 2004;22:1807-14.
3. Meeker AK, Argani P. Telomere shortening occurs early during breast tumorigenesis: a cause of chromosome destabilization underlying malignant transformation? J Mammary Gland Biol Neoplasia 2004;9:285-96.
4. Damle RN, Batliwalla FM, Ghiotto F, et al. Telomere length and telomerase activity delineate distinctive replicative features of the B-CLL subgroups defined by immunoglobulin V gene mutations. Blood 2004;103:375-82.
5. Grabowski P, Hultdin M, Karlsson K, et al. Telomere length as a prognostic parameter in chronic lymphocytic leukemia with special reference to VH gene mutation status. Blood 2005;105:4807-12.
6. Rossi D, Lobetti Bodoni C, Genuardi E, et al. Telomere length is an independent predictor of survival, treatment requirement and Richter's syndrome transformation in chronic lymphocytic leukemia. Leukemia 2009;23:1062-72.
7. Sellmann L, de Beer D, Bartels M, et al. Telomeres and prognosis in patients with chronic lymphocytic leukaemia. Int J Hematol 2011;93:74-82.
8. Roos G, Krober A, Grabowski P, et al. Short telomeres are associated with genetic complexity, high risk genomic aberrations, and short survival in chronic lymphocytic leukemia. Blood 2008; 111:2246-52.
9. Ricca I, Rocci A, Drandi D, et al. Telomere length identifies two different prognostic subgroups among VH-unmutated B-cell chronic lymphocytic leukemia patients. Leukemia 2007; 21:697-705.
10. Chin K, de Solorzano CO, Knowles D, et al. In situ analyses of genome instability in breast cancer. Nat Genet. 2004; 36:984-8.
11. Heaphy CM, Baumgartner KB, Bisoffi M, Baumgartner RN, Griffith JK. Telomere DNA content predicts breast cancer-free survival interval. Clin Cancer Res. 2007; 13:7037-43.
12. Lu L, Zhang C, Zhu G, et al. Telomerase Expression and Telomere Length in Breast Cancer and their Associations with Adjuvant Treatment and Disease Outcome. Breast Cancer Res. 2011; 13:R56.
13. Aubert G, Hills M, Lansdorp PM. Telomere length measurement-Caveats and a critical assessment of the available technologies and tools. Mutat Res. 2011 Jun 8 [Epib ahead of print].
14. Baird DM. New developments in telomere length analysis. Exp Gerontol. 2005; 40:363-8.
15. Lin TT, Letsolo BT, Jones RE, et al. Telomere dysfunction and fusion during the progression of chronic lymphocytic leukaemia: evidence for a telomere crisis. Blood 2010; 116:1899-907.
16. Capper R, Britt-Compton B, Tankimanova M, et al. The nature of telomere fusion and a definition of the critical telomere length in human cells. Genes Dev. 2007; 21:2495-508.
17. Letsolo BT, Rowson J, Baird DM. Fusion of short telomeres in human cells is characterised by extensive deletion and microhomology and can result in complex rearrangements. Nucleic Acids Res. 2010; 38:1841-52.
18. Cawthon, R.M. Telomere measurement by quantitative PCR. Nucleic Acids Res. 2002; 30; e47.
19. Shen, J., Terry, M.B., Gurvich, I., Liao, Y., Senie, R.T. and Santella, R.M. Short telomere length and breast cancer risk: a study in sister sets. Cancer Res. 2007; 67: 5538-5544. Reviewed in Aviv, A. The epidemiology of human telomeres: faults and promises. J Gerontol A Biol Sci Med Sci, 2008; 63: 979-983.
20. Baird DM, Rowson J, Wynford-Thomas D, Kipling D. Extensive allelic variation and ultrashort telomeres in senescent human cells. Nat Genet. 2003; 33:203-7.
21. Britt-Compton B, Rowson J, Locke M, Mackenzie I, Kipling D, Baird DM. Structural stability and chromosome-specific telomere length is governed by cis-acting determinants in humans. Hum Mol Genet. 2006; 15:725-33.
22. Baird DM, Britt-Compton B, Rowson J, Amso NN, Gregory L, Kipling D. Telomere instability in the male germline. Hum Mol Genet. 2006; 15:45-51.
23. Britt-Compton B, Capper R, Rowson J, Baird DM. Short telomeres are preferentially elongated by telomerase in human cells. FEBS Lett 2009; 583:3076-80.
24. Binet JL, Auquier A, Dighiero G, et al. A new prognostic classification of chronic lymphocytic leukemia derived from a multivariate survival analysis. Cancer 1981; 48: 198-206.
25. Hewamana S, Alghazal S, Lin TT, et al. The NF-kappaB subunit Rel A is associated with in vitro survival and clinical disease progression in chronic lymphocytic leukemia and represents a promising therapeutic target. Blood 2008; 111:4681-9.
26. Sambrook J, Fritsh EF, Maniatis T. Molecular cloning: a laboratory manual. 2nd Edition ed. New York: Cold spring harbour laboratory press; 1989.

**Table-1. Upper limit and mean telomere length at which telomere end-end fusion events can be detected for five different chromosome ends.**

| Chromosome end | Upper limit (kb) | Mean of fusogenic range (kb) |
|---|---|---|
| XpYp | 3.81 | 2.26 |
| 17p | 4.81 | 2.57 |
| 2p | 5.01 | 3.01 |
| 16p | 4.49 | 2.94 |
| 18q | 4.47 | 2.66 |
| | | |
| Mean ± SD | 4.52 ±0.46 | 2.69 ± 0.30 |

**Table-2. Comparison of prognostic factors in univariate analysis in terms of time to first treatment and overall survival**

| Parameter | Time to first treatment | | | Overall survival | | |
|---|---|---|---|---|---|---|
| | Median (years) | HR (95% CI) | P-value | Median (years) | HR (95%CI) | P-value |
| TL (fusion mean) | | 23.2 (11.0-48.7) | <0.0001 | | 71.3 (20.0.253.7) | <0.0001 |
| ≤2.26kb | 1.8 | | | 7.5 | | |
| >2.26kb | not reached | | | not reached | | |
| *IGHV* status | | 4.6 (2.4-8.8) | <0.0001 | | 2.9 (1.0-8.1) | 0.04 |
| ≥98% | 2.9 | | | not reached | | |
| <98% | not reached | | | not reached | | |
| CD38 | | 3.0 (1.7-5.3) | 0.0003 | | 3.2 (1.1-9.1) | 0.03 |
| ≥20% | 3.0 | | | not reached | | |
| <20% | not reached | | | not reached | | |
| ZAP-70 | | 1.7 (1.0-2.9) | 0.07 | | 2.3 (0.9-6.2) | 0.08 |
| ≥20% | 6.0 | | | not reached | | |
| <20% | not reached | | | not reached | | |
| β2-M | | 3.1 (1.6-6.2) | 0.001 | | 3.1 (0.99-9.5) | 0.05 |
| ≥4 g/dL | 3.0 | | | 12.7 | | |
| <4g/dL | Not reached | | | Not reached | | |
| Genetics | | 7.7 (3.1-18.9) | <0.0001 | | 10.1 (2.7-30.8) | 0.0004 |
| 11q⁻/17p⁻ | 2.0 | | | 6.9 | | |
| N/O | not reached | | | not reached | | |

| | | | | | | |
|---|---|---|---|---|---|---|
| TL (fusion mean): The mean telomere length of the samples in which fusion events were detected *IGHV* status: <98% sequence homology with the closet germline sequence (mutated); ≥98% sequence homology with the closest germline sequence (unmutated) β2-M: beta 2 microglobulin 11q⁻ and 17p⁻: any FISH or karyotypic abnormality of 11q or 17p N: No detectable cytogenetic aberration by FISH O**:** Other cytogenetic abnormality (excluding 11q⁻ or 17p⁻) HR = Hazard ratio 95% CI = 95% confidence interval | | | | | | |

**Table-3. Clinical characteristics of the 184 CLL patient cohort.**

| Factor | Subset | Number |
|---|---|---|
| Median Age | | 64 years |
| Range | | 27 - 95 years |
| Median Follow up | | 5.8 years |
| Required treatment | Treated | 75 |
| | Untreated | 104 |
| CD38 | <20% | 105 |
| | ≥20% | 53 |
| | Not Determined | 26 |
| Genetics | 11q⁻/17p⁻ | 21 |
| | N/O | 125 |
| | Not Determined | 38 |
| *IGHV* Status | <98% | 93 |
| | ≥98% | 45 |
| | Not Determined | 46 |
| ZAP-70 | <20% | 95 |
| | ≥20% | 59 |
| | Not Determined | 30 |
| β2-microglobulin | <4 mg/dL | 81 |
| | ≥4 mg/dL | 37 |
| | Not Determined | 56 |

**Table-4. Analysis of concordant datasets combining telomere length with known prognostic markers.**

| Parameter | n | TTFT | | **OS** | |
|---|---|---|---|---|---|
| | | HR (95% CI) | P-value | HR (95% CI) | P-value |
| TL + *IGHV* | | 141.2 (40.5-492.1) | <0.0001 | 72.4 (14.4-365.5) | <0.0001 |
| TL≤2.26kb + UM *IGHV* | 16 | | | | |
| TL>2.26kb + M *IGHV* | 79 | | | | |
| TL + CD38 | | 134.7 (35.5-512.1) | <0.0001 | 2915 (261.1-32550) | <0.0001 |
| TL≤2.26kb + CD38⁺ | 14 | | | | |
| TL>2.26kb + CD38⁻ | 88 | | | | |
| TL + ZAP-70 | | 29.3 (9.9-86.0) | <0.0001 | 681.2 (99.9-4645) | <0.0001 |
| TL≤2.26kb + ZAP-70⁺ | 16 | | | | |
| TL>2.26kb + ZAP-70⁻ | 80 | | | | |
| TL + cytogenetics | | 52.2 (14.8-186.3) | <0.0001 | 308.5 (44.1-2147) | <0.0001 |
| TL≤2.26kb + 11q-/17p- | 13 | | | | |
| TL>2.26kb + Other | 105 | | | | |

| | | | | | |
|---|---|---|---|---|---|
| *Analysis is shown for concordant cases only e.g. TL≤2.26kb / *IGHV* unmutated vs TL>2.26kb / *IGHV* mutated. Discordant datasets were not included in this analysis. TL = telomere length UM = *IGHV* unmutated cases: ≥98% sequence homology with the closest germline sequence M *= IGHV* mutated cases: <98% sequence homology with the closest germline sequence | | | | | |

## Claims

1. A prognostic method for determining the progression of a disease or condition including or **characterised by** telomere shortening comprising:
i) using high-resolution telomere length analysis to determine the longest mean telomere length at which telomere end-end fusion events can be detected in samples of tissue from a number of individuals presenting with the same disease or condition, in order to identify a threshold value that represents an indication of the mean telomere length at which telomeres become dysfunctional and capable of fusion ;
ii) determining the prognostic mean telomere length of samples of tissue from a number of individuals presenting with said disease or condition, by taking those samples whose mean telomere length is less than said threshold value and averaging the mean telomere length of those samples;
iii) determining the mean test telomere length of a sample taken from a patient suspected of having or presenting with said disease or condition and, where said mean test telomere length is less than said prognostic mean telomere length it indicates that time to first treatment is poor and/or response to treatment is poor and/or overall survival is poor; or
iv) determining the mean test telomere length of a sample taken from a patient suspected of having or presenting with said disease or condition and, where said mean test telomere length is greater than said prognostic mean telomere length it indicates that time to first treatment is good and/or response to treatment is good and/or overall survival is good.

2. The method according to claim 1 wherein said fusion event in part i) above is verified as being such by direct DNA sequence analysis.

3. The method according to claim 1 or 2 wherein, additionally or alternatively, said prognostic mean telomere length of samples of tissue from a number of individuals presenting with said disease is determined by taking those samples that exhibit telomere fusion and averaging the mean telomere length of those samples.

4. The method according to any preceding claim wherein said disease or condition is one of the following diseases: aging, alzheimer's disease; brain infarction; heart disease; chronic HIV infection; chronic hepatitis; skin diseases; chronic inflammatory bowel disease; ulcerative colitis; anaemia; atherosclerosis; Barrett's oesophagus and cancer, including pre-cancerous conditions.

5. The method according to claim 4 wherein said cancer is either a haematological malignancy or a solid tumour.

6. The method according to claim 5 wherein said cancer is CLL, MDS or breast cancer.

7. The method according to any preceding claim wherein said telomere length at which telomere end-end fusion events can be detected is determined for a single chromosome.

8. The method according to claims 1-6 wherein said telomere length at which telomere end-end fusion events can be detected is determined for a number of different chromosomes.

9. The method according to claim 8 wherein the average upper limit for detecting end-end fusion events in different chromosomes is used in part i) of claim 1 and the average mean telomere length in the fusogenic range for these different chromosomes is used in part ii) of claim 1.

10. A prognostic method for determining the progression of a disease or condition including or **characterized by** telomere shortening comprising:
i) using high-resolution telomere length analysis to determine the prognostic mean telomere length of samples of tissue from a number of individuals presenting with said disease or condition, whose mean telomere length, the value of which has been determined according to claim 1, is less than a 4.52kb telomere length threshold value at which telomere end-end fusion events can be detected in cancerous disease, by taking those samples whose mean telomere length is less than said threshold value and averaging the mean telomere length of those samples;
ii) determining the mean test telomere length of a sample taken from a patient suspected of having or presenting with said disease or condition and, where said mean test telomere length is less than said prognostic mean telomere length it indicates that the time to first treatment is poor and/or the response to treatment is poor and/or overall survival is poor; or
iii) determining the mean test telomere length of a sample taken from a patient suspected of having or presenting with said disease or condition and, where said mean test telomere length is greater than said prognostic mean telomere length it indicates that time to first treatment is good and/or response to treatment is good and/or overall survival is good.

11. The method according to claim 10 wherein said disease is a cancer such as CLL, breast cancer or MDS.

12. The method according to claim 11 wherein said prognostic mean telomere length is 2.26kb.

13. The method according to claim 10 or 11 wherein said telomere length at which telomere end-end fusion events can be detected is determined for a number of different chromosomes.

14. The method according to claim 13 wherein the chromosomes are XpYp, 17p, 2p, 16p and 18q.

15. The method according to any one of claim 10 - 14 wherein in part i), additionally or alternatively, said prognostic mean telomere length of samples of tissue from a number of individuals presenting with said disease or condition is determined by taking those samples that exhibit telomere fusion and averaging the mean telomere length of those samples.

16. A prognostic method for determining the progression of a disease or condition including or **characterized by** telomere shortening comprising:
i) determining the mean test telomere length of a sample taken from a patient suspected of having or presenting with said disease or condition and, where said mean test telomere length is less than a prognostic mean telomere length of 2.69kb, the value of which has been determined according to claim 1, it indicates that the time to first treatment is poor and/or the response to treatment is poor and/or overall survival is poor; or
ii) determining the mean test telomere length of a sample taken from a patient suspected of having or presenting with said disease or condition and, where said mean test telomere length is greater than a prognostic mean telomere length of 2.69kb, the value of which has been determined according to claim 1, it indicates that the time to first treatment is good and/or the response to treatment is good and/or overall survival is good.

17. The method according to claim 16 wherein said disease is a haematological cancer such as CLL or MDS.

18. The method according to claim 17 wherein said prognostic mean telomere length is 2.26kb.

19. The method according to claims 15-18 wherein said prognostic mean telomere length is determined for a number of different chromosomes.

20. The method according to claim 19 wherein the chromosomes are XpYp, 17p, 2p, 16p and 18q.

21. The method according to any one of claims 16 - 20 wherein in part i), additionally or alternatively, said prognostic mean telomere length of samples of tissue from a number of individuals presenting with said disease is determined by taking those samples that exhibit telomere fusion and averaging the mean telomere length of those samples.

## Patentansprüche

1. Prognosemethode zur Bestimmung der Progression einer Erkrankung oder gesundheitlichen Störung, einschließlich oder charakterisiert durch Telomerverkürzung, umfassend:
i) Verwenden einer hochauflösenden Telomer-Längenanalyse zur Bestimmung der längsten durchschnittlichen Telomerlänge, bei der Fusionen der Telomerenden in Gewebeproben mehrerer Individuen mit der gleichen Erkrankung oder gesundheitlichen Störung nachgewiesen werden können, um einen Schwellenwert zu identifizieren, der der mittleren Telomerlänge entspricht, bei der Telomere dysfunktionell werden und fusionieren können;
ii) Bestimmen der prognostischen mittleren Telomerlänge von Gewebeproben mehrerer Individuen, die von der Erkrankung oder gesundheitlichen Störung befallen sind, indem die Proben genommen werden, deren mittlere Telomerlänge unter dem Schwellenwert liegt und die mittlere Telomerlänge dieser Proben gemittelt wird;
iii) Bestimmen der mittleren Test-Telomerlänge einer Probe von einem Patienten, bei dem der Verdacht besteht, die Erkrankung oder gesundheitliche Störung zu haben oder von dieser befallen zu sein, wobei, falls die mittlere Test-Telomerlänge kürzer als die prognostische mittlere Telomerlänge ist, dies anzeigt, dass die Zeit bis zur ersten Behandlung gering ist und/oder die Reaktion auf die Behandlung gering ist und/oder das Gesamtüberleben gering ist; oder
iv) Bestimmen der mittleren Test-Telomerlänge einer Probe von einem Patienten, bei dem der Verdacht besteht, die Erkrankung oder gesundheitliche Störung zu haben oder davon befallen zu sein, wobei, falls die mittlere Test-Telomerlänge länger als die prognostische mittlere Telomerlänge ist, dies anzeigt, dass die Zeit für die erste Behandlung gut ist und/oder die Reaktion auf die Behandlung gut ist und/oder das Gesamtüberleben gut ist.

2. Methode gemäß Anspruch 1, wobei das Fusionsereignis im Teil i) oben als solches durch direkte DNA-Sequenzanalyse verifiziert wird.

3. Methode gemäß Anspruch 1 oder 2, wobei zusätzlich oder alternativ die prognostische mittlere Telomerlänge der Gewebeproben mehrerer Individuen, die von der Erkrankung befallen sind, durch Verwendung dieser Proben bestimmt wird, die eine Telomerfusion zeigen und durch Mittelung der mittleren Telomerlänge dieser Proben.

4. Methode gemäß einem vorhergehenden Anspruch, wobei die Erkrankung oder gesundheitliche Störung eine der folgenden Erkrankungen ist: Altern, Alzheimersche Krankheit; Hirninfarkt; Herzerkrankung; chronische HIV-Infektion; chronische Hepatitis; Hauterkrankungen; chronisch entzündliche Darmerkrankung, Colitis ulcerosa; Anämie; Atherosklerose; Barrett-Ösophagus und Krebs, einschließlich Krebsvorstufen.

5. Methode gemäß Anspruch 4, wobei die Krebserkrankung entweder eine hämatologische Malignität oder ein solider Tumor ist.

6. Methode gemäß Anspruch 5, wobei die Krebserkrankung entweder CLL, MDS oder Brustkrebs ist.

7. Methode gemäß einem vorhergehenden Anspruch, wobei die Telomerlänge, bei der die Fusion der Telomerenden nachgewiesen werden kann, für ein einzelnes Chromosom bestimmt wird.

8. Methode gemäß einem der Ansprüche 1-6, wobei die Telomerlänge, bei der die Fusion der Telomerenden nachgewiesen werden kann, für mehrere verschiedene Chromosomen bestimmt wird.

9. Methode gemäß Anspruch 8, wobei der mittlere obere Grenzwert für den Nachweis der Fusion der Enden in verschiedenen Chromosomen in Teil i) von Anspruch 1 verwendet wird und die durchschnittliche mittlere Telomerlänge im fusogenen Bereich für diese unterschiedlichen Chromosomen in Teil ii) von Anspruch 1 verwendet wird.

10. Prognosemethode zur Bestimmung der Progression einer Erkrankung oder gesundheitlichen Störung einschließlich oder charakterisiert durch Telomerverkürzung, umfassend:
i) Verwenden einer hochauflösenden Telomer-Längenanalyse zur Bestimmung der prognostischen durchschnittlichen Telomerlänge der Gewebeproben mehrerer Individuen, die von der Erkrankung oder gesundheitlichen Störung befallen sind, deren mittlere Telomerlänge, dem Wert, der gemäß Anspruch 1 bestimmt wurde, kleiner ist als ein Schwellenwert von 4,52 kb für die Telomerlänge, bei der die Fusion von Telomerenden bei Krebserkrankungen nachgewiesen werden kann, indem diese Proben genommen werden, deren mittlere Telomerlänge kürzer ist als der Schwellenwert und die mittlere Telomerlänge dieser Proben gemittelt wird;
ii) Bestimmen der mittleren Test-Telomerlänge einer Probe von einem Patienten, bei dem der Verdacht besteht, die Erkrankung oder gesundheitliche Störung zu haben oder davon befallen zu sein, wobei die mittlere Test-Telomerlänge kürzer als die prognostische mittlere Telomerlänge ist, die anzeigt, dass die Zeit bis zur ersten Behandlung gering ist und/oder die Reaktion auf die Behandlung gering ist und/oder das Gesamtüberleben gering ist; oder
iii) Bestimmen der mittleren Test-Telomerlänge einer Probe von einem Patienten, bei dem der Verdacht besteht, die Erkrankung oder gesundheitliche Störung zu haben oder davon befallen zu sein, und, wobei die mittlere Test-Telomerlänge länger ist als die prognostische mittlere Telomerlänge, die anzeigt, dass die Zeit für die erste Behandlung gut ist und/oder die Reaktion auf die Behandlung gut ist und/oder das Gesamtüberleben gut ist.

11. Methode gemäß Anspruch 10, wobei die Erkrankung eine Krebserkrankung ist, wie beispielsweise CLL, Brustkrebs oder MDS.

12. Methode gemäß Anspruch 11, wobei die prognostische mittlere Telomerlänge 2,26 kb beträgt.

13. Methode gemäß Anspruch 10 oder 11, wobei die Telomerlänge, bei der die Fusion der Telomerenden nachgewiesen werden kann, für mehrere verschiedene Chromosomen bestimmt wird.

14. Methode gemäß Anspruch 13, wobei die Chromosomen XpYp, 17p, 2p, 16p und 18q sind.

15. Methode gemäß Anspruch 10 - 14, wobei in Teil i) zusätzlich oder alternativ die prognostische mittlere Telomerlänge der Gewebeproben mehrerer Individuen, die von der Erkrankung oder gesundheitlichen Störung befallen sind, durch Verwendung derjenigen Proben bestimmt wird, die eine Telomerfusion zeigen und durch Mittelung der mittleren Telomerlänge dieser Proben.

16. Prognosemethode zur Bestimmung der Progression einer Erkrankung oder gesundheitlichen Störung einschließlich oder charakterisiert durch Telomerverkürzung, umfassend:
i) Bestimmen der mittleren Test-Telomerlänge einer Probe von einem Patienten, bei dem der Verdacht besteht, die Erkrankung oder gesundheitliche Störung zu haben oder davon befallen zu sein, wobei die mittlere Test-Telomerlänge kürzer als eine prognostische mittlere Telomerlänge von 2,69 kb ist, dem Wert, der gemäß Anspruch 1 bestimmt wurde, die anzeigt, dass die Zeit bis zur ersten Behandlung gering ist und/oder die Reaktion auf die Behandlung gering ist und/oder das Gesamtüberleben gering ist; oder
ii)Bestimmen der mittleren Test-Telomerlänge einer Probe von einem Patienten, bei dem der Verdacht besteht, die Erkrankung oder gesundheitliche Störung zu haben oder davon befallen zu sein, wobei die mittlere Test-Telomerlänge größer als eine prognostische mittlere Telomerlänge von 2,69 kb ist, dem Wert, der gemäß Anspruch 1 bestimmt wurde, die anzeigt, dass die Zeit bis zur ersten Behandlung gut ist und/oder die Reaktion auf die Behandlung gut ist und/oder das Gesamtüberleben gut ist.

17. Methode gemäß Anspruch 16, wobei die Erkrankung ein Blutkrebs ist, wie beispielsweise CLL oder MDS.

18. Methode gemäß Anspruch 17, wobei die prognostische mittlere Telomerlänge 2,26 kb beträgt.

19. Methode gemäß Ansprüchen 15-18, wobei die prognostische mittlere Telomerlänge für mehrere verschiedene Chromosomen bestimmt wird.

20. Methode gemäß Anspruch 19, wobei die Chromosomen XpYp, 17p, 2p, 16p und 18q sind.

21. Methode gemäß einem der Ansprüche 16 - 20, wobei in Teil i) zusätzlich oder alternativ die prognostische mittlere Telomerlänge der Gewebeproben mehrerer Individuen, die von der Erkrankung befallen sind, durch Verwendung dieser Proben bestimmt wird, die eine Telomerfusion zeigen und durch Mittelung der mittleren Telomerlänge dieser Proben.

## Revendications

1. Procédé de pronostic pour déterminer la progression d'une maladie ou d'une affection comprenant ou **caractérisée par** un raccourcissement des télomères comprenant:
i) l'utilisation d'une analyse de la longueur des télomères à haute résolution pour déterminer la longueur moyenne des télomères la plus longue à laquelle des événements de fusion d'extrémité à extrémité des télomères peuvent être détectés dans des échantillons de tissus provenant d'un certain nombre d'individus présentant la même maladie ou affection, afin d'identifier une valeur seuil qui représente une indication de la longueur moyenne des télomères à laquelle les télomères deviennent dysfonctionnels et capables de fusionner;
ii) la détermination de la longueur moyenne des télomères pronostique des échantillons de tissus provenant d'un certain nombre d'individus présentant ladite maladie ou affection, en prenant ces échantillons dont la longueur moyenne des télomères est inférieure à ladite valeur seuil et en calculant la moyenne de la longueur moyenne des télomères de ces échantillons;
iii) la détermination de la longueur moyenne des télomères à tester d'un échantillon prélevé chez un patient supposé porteur de ou présentant une maladie ou une affection et, où si ladite longueur moyenne des télomères à tester est inférieure à ladite longueur moyenne des télomères pronostique, cela indique que le temps jusqu'au premier traitement est faible et/ou que la réponse au traitement est faible et/ou que la survie globale est faible; ou
iv) la détermination de la longueur moyenne des télomères à tester d'un échantillon prélevé chez un patient supposé porteur de ou présentant ladite maladie ou affection et, où si ladite longueur moyenne des télomères à tester est supérieure à ladite longueur moyenne des télomères pronostique, cela indique que le temps jusqu'au premier traitement est bon et/ou que la réponse au traitement est bonne et/ou que la survie globale est bonne.

2. Procédé selon la revendication 1, dans lequel ledit événement de fusion dans la partie i) ci-dessus est vérifié comme étant tel par une analyse directe de la séquence d'ADN.

3. Procédé selon la revendication 1 ou 2, dans lequel, en outre ou en variante, ladite longueur moyenne des télomères pronostique des échantillons de tissus provenant d'un certain nombre d'individus présentant ladite maladie est déterminée en prenant ces échantillons qui affichent une fusion des télomères et en calculant la moyenne de la longueur moyenne des télomères de ces échantillons.

4. Procédé selon l'une quelconque des revendications précédentes, dans lequel ladite maladie ou affection est l'une des maladies suivantes: le vieillissement, la maladie d'Alzheimer; l'infarctus cérébral; les cardiopathies; l'infection chronique par le VIH; l'hépatite chronique; les maladies cutanées; les maladies inflammatoires chroniques des intestins; la rectocolite hémorragique; l'anémie; l'athérosclérose; l'oesophage de Barrett et le cancer, y compris les affections précancéreuses.

5. Procédé selon la revendication 4, dans lequel ledit cancer est soit une affection maligne hématologique soit une tumeur solide.

6. Procédé selon la revendication 5, dans lequel ledit cancer est la LLC, les SMD ou le cancer du sein.

7. Procédé selon l'une quelconque des revendications précédentes, dans lequel ladite longueur des télomères à laquelle des événements de fusion d'extrémité à extrémité des télomères peuvent être détectés est déterminée pour un seul chromosome.

8. Procédé selon les revendications 1 à 6, dans lequel ladite longueur des télomères à laquelle des événements de fusion d'extrémité à extrémité des télomères peuvent être détectés est déterminée pour un certain nombre de chromosomes différents.

9. Procédé selon la revendication 8, dans lequel la limite supérieure moyenne pour la détection des événements de fusion d'extrémité à extrémité dans différents chromosomes est utilisée dans la partie i) de la revendication 1 et la moyenne de la longueur moyenne des télomères dans la plage fusogène pour ces différents chromosomes est utilisée dans la partie ii) de la revendication 1.

10. Procédé de pronostic pour déterminer la progression d'une maladie ou d'une affection comprenant ou **caractérisée par** un raccourcissement des télomères comprenant:
i) l'utilisation d'une analyse de la longueur des télomères à haute résolution pour déterminer la longueur moyenne des télomères pronostique des échantillons de tissus provenant d'un certain nombre d'individus présentant ladite maladie ou affection, dont la longueur moyenne des télomères, dont la valeur a été déterminée selon la revendication 1, est inférieure à une valeur seuil de longueur de télomère de 4,52 kb à laquelle des événements de fusion d'extrémité à extrémité des télomères peuvent être détectés dans une maladie cancéreuse, en prenant ces échantillons dont la longueur moyenne des télomères est inférieure à ladite valeur seuil et en calculant la moyenne de la longueur moyenne des télomères de ces échantillons;
ii) la détermination de la longueur moyenne des télomères à tester d'un échantillon prélevé chez un patient supposé porteur de ou présentant ladite maladie ou affection et, où si ladite longueur moyenne des télomères à tester est inférieure à ladite longueur moyenne des télomères pronostique, cela indique que le temps jusqu'au premier traitement est faible et/ou que la réponse au traitement est faible et/ou que la survie globale est faible; ou
iii) la détermination de la longueur moyenne des télomères à tester d'un échantillon prélevé chez un patient supposé porteur de ou présentant ladite maladie ou affection et, où si ladite longueur moyenne des télomères à tester est supérieure à ladite longueur moyenne des télomères pronostique, cela indique que le temps jusqu'au premier traitement est bon et/ou que la réponse au traitement est bonne et/ou que la survie globale est bonne.

11. Procédé selon la revendication 10, dans lequel ladite maladie est un cancer tel que la LLC, le cancer du sein ou les SMD.

12. Procédé selon la revendication 11, dans lequel ladite longueur moyenne des télomères pronostique est de 2,26 kb.

13. Procédé selon la revendication 10 ou 11, dans lequel ladite longueur des télomères à laquelle des événements de fusion d'extrémité à extrémité des télomères peuvent être détectés est déterminée pour un certain nombre de chromosomes différents.

14. Procédé selon la revendication 13, dans lequel les chromosomes sont XpYp, 17p, 2p, 16p et 18q.

15. Procédé selon l'une quelconque des revendications 10 à 14, dans lequel dans la partie i), en outre ou en variante, ladite longueur moyenne des télomères pronostique des échantillons de tissus provenant d'un certain nombre d'individus présentant ladite maladie ou affection est déterminée en prenant ces échantillons qui affichent une fusion des télomères et en calculant la moyenne de la longueur moyenne des télomères de ces échantillons.

16. Procédé de pronostic pour déterminer la progression d'une maladie ou d'une affection comprenant ou **caractérisée par** un raccourcissement des télomères comprenant:
i) la détermination de la longueur moyenne des télomères à tester d'un échantillon prélevé chez un patient supposé porteur de ou présentant ladite maladie ou affection et, où si ladite longueur moyenne des télomères à tester est inférieure à une longueur moyenne des télomères pronostique de 2,69 kb, dont la valeur a été déterminée selon la revendication 1, cela indique que le temps jusqu'au premier traitement est faible et/ou de la réponse au traitement est faible et/ou que la survie globale est faible; ou
ii) la détermination de la longueur moyenne des télomères à tester d'un échantillon prélevé chez un patient supposé porteur de ou présentant ladite maladie ou affection et, où si ladite longueur moyenne des télomères à tester est supérieure à une longueur moyenne des télomères pronostique de 2,69 kb, dont la valeur a été déterminée selon la revendication 1, cela indique que le temps jusqu'au premier traitement est bon et/ou que la réponse au traitement est bonne et/ou que la survie globale est bonne.

17. Procédé selon la revendication 16, dans lequel ladite maladie est un cancer hématologique tel que la LLC ou les SMD.

18. Procédé selon la revendication 17, dans lequel ladite longueur moyenne des télomères pronostique est de 2,26 kb.

19. Procédé selon les revendications 15 à 18, dans lequel ladite longueur moyenne des télomères pronostique est déterminée pour un certain nombre de chromosomes différents.

20. Procédé selon la revendication 19, dans lequel les chromosomes sont XpYp, 17p, 2p, 16p et 18q.

21. Procédé selon l'une quelconque des revendications 16 à 20, dans lequel dans la partie i), en outre ou en variante, ladite longueur moyenne des télomères pronostique des échantillons de tissus provenant d'un certain nombre d'individus présentant ladite maladie est déterminée en prenant ces échantillons qui affichent une fusion des télomères et en calculant la moyenne de la longueur moyenne des télomères de ces échantillons.


### SEQUENCE LISTING

<110> University University College Cardiff Consultants Limited Baird, Duncan Pepper, Christopher Fegan, Christopher Fegan, Christopher
<120> Telomere Length and Disease
<130> 0043P/WO
<150> GB1113968.0
   <151> 2011-08-15
<160> 23
<170> PatentIn version 3.5
<210> 1
   <211> 20
   <212> DNA
   <213> Homo sapiens
<400> 1
   accaggtttt ccagtgtgtt 20
<210> 2
   <211> 20
   <212> DNA
   <213> Homo sapiens
<400> 2
   ggctgaacta tagcctctgc 20
<210> 3
   <211> 20
   <212> DNA
   <213> Homo sapiens
<400> 3
   cttggtgtcg agagaggtag 20
<210> 4
   <211> 20
   <212> DNA
   <213> Homo sapiens
<400> 4
   cctgtaacgc tgttaggtac 20
<210> 5
   <211> 20
   <212> DNA
   <213> Homo sapiens
<400> 5
   cctggcatgg tattgacatg 20
<210> 6
   <211> 20
   <212> DNA
   <213> Homo sapiens
<400> 6
   tggtcttata cactgtgttc 20
<210> 7
   <211> 25
   <212> DNA
   <213> Homo sapiens
<400> 7
   agctagctat ctactctaac agagc 25
<210> 8
   <211> 20
   <212> DNA
   <213> Homo sapiens
<400> 8
   tctgaaagtg gaccaatcag 20
<210> 9
   <211> 20
   <212> DNA
   <213> Homo sapiens
<400> 9
   tctgaaagtg gacctatcag 20
<210> 10
   <211> 21
   <212> DNA
   <213> Homo sapiens
<400> 10
   agaatcctgt cctcaacaag t 21
<210> 11
   <211> 19
   <212> DNA
   <213> Homo sapiens
<400> 11
   ttgtctcagg gtcctagtg 19
<210> 12
   <211> 20
   <212> DNA
   <213> Homo sapiens
<400> 12
   ggttatcaac caggtgctct 20
<210> 13
   <211> 20
   <212> DNA
   <213> Homo sapiens
<400> 13
   ggttatcgac caggtgctcc 20
<210> 14
   <211> 21
   <212> DNA
   <213> Homo sapiens
<400> 14
   tgtgtctgga attggtgggt t 21
<210> 15
   <211> 23
   <212> DNA
   <213> Homo sapiens
<400> 15
   cctagtgtgt ctggaattgg ttc 23
<210> 16
   <211> 21
   <212> DNA
   <213> Homo sapiens
<400> 16
   cagggaccgg gacaaataga c 21
<210> 17
   <211> 24
   <212> DNA
   <213> Homo sapiens
<400> 17
   gaatccacgg attgctttgt gtac 24
<210> 18
   <211> 22
   <212> DNA
   <213> Homo sapiens
<400> 18
   gtgaataatc aaggtcagag ca 22
<210> 19
   <211> 23
   <212> DNA
   <213> Homo sapiens
<400> 19
   cctgtgggtc taaaaccaga agg 23
<210> 20
   <211> 21
   <212> DNA
   <213> Homo sapiens
<400> 20
   gagctgcgtt ttgctgagca c 21
<210> 21
   <211> 24
   <212> DNA
   <213> Homo sapiens
<400> 21
   cagaccttgg aggcacggcc ttcg 24
<210> 22
   <211> 20
   <212> DNA
   <213> Homo sapiens
<400> 22
   gggagatcca caccgtagca 20
<210> 23
   <211> 20
   <212> DNA
   <213> Homo sapiens
<400> 23
   acagcctttt ggggtaccgc 20